# EUROPEAN PATENT APPLICATION

(11) **EP 3 330 375 A1**
(43) Date of publication of application: **06.06.2018**
(21) Application number: 15763639.0
(22) Date of filing: 29.07.2015
(51) Int. Cl.: C12N 9/24, C12P 19/14

(54) **EXPRESSION OF RECOMBINANT BETA-XYLOSIDASE ENZYMES**

(71) Applicant: Abengoa Bioenergía Nuevas Tecnologías, S. A., 41014 Sevilla (ES)
(72) Inventor: DÍEZ GARCÍA, Bruno, 41014 Sevilla (ES); VALBUENA CRESPO, Noelia, 41014 Sevilla (ES); REYES SOSA, Francisco Manuel, 41014 Sevilla (ES); MORENO PÉREZ, Antonio Javier, 41014 Sevilla (ES); PÉREZ GÓMEZ, Dolores, 41014 Sevilla (ES); MARTÍN PÉREZ, Lucía, 41014 Sevilla (ES); GAVALDÁ MARTÍN, Sandra, 41014 Sevilla (ES); PLATERO GÓMEZ, Ana Isabel, 41014 Sevilla (ES); VIÑAS DE LA CRUZ, Laura, E-46980 Paterna (València) (ES); SÁNCHEZ ZAMORANO, Laura, 41014 Sevilla (ES); ÁLVAREZ NÚÑEZ, Consolación, 41014 Sevilla (ES); BERMÚDEZ ALCÁNTARA, María de los Ángeles, 41014 Sevilla (ES); LEDESMA GARCÍA, Laura, 41014 Sevilla (ES); ROCHA MARTÍN, Javier, 41014 Sevilla (ES); RAMOS MARTÍN, Juan Luis, 41014 Sevilla (ES); BENÍTEZ CASANOVA, Laura, 41014 Sevilla (ES); LÓPEZ MORALES, Macarena, 41014 Sevilla (ES)
(74) Representative: Pons
(86) International application number: PCT/ES2015/070589
(87) International publication number: WO 2017/017292

(57) **Abstract**

The present invention relates to a *Myceliophthora thermophila* cell, which expresses a nucleotide sequence that codifies a recombinant beta-xylosidase enzyme comprising an amino-acid sequence having at least 70% identity with SEQ ID NO: 1, an enzymatic composition comprising said cell and/or the recombinant enzyme with beta-xylosidase activity expressed by said cell, the use of this host cell, the recombinant enzyme with beta-xylosidase activity expressed by said cell or the composition for the degradation of biomass, and a method of producing biological products, preferably bioethanol, comprising the use of said host cell, the recombinant enzyme with the beta-xylosidase activity expressed by said cell or said composition.

SEQUENCE LISTING

<110> ABENGOA BIOENERGÍA NUEVAS TECNOLOGÍAS, S.A.

<120> EXPRESSION OF RECOMBINANT BETA-XYLOSIDASE ENZYMES

<130> PCT1861.60

<160> 11

<170> PatentIn version 3.5

<210> 1
<211> 740
<212> PRT
<213> Aspergillus nidulans

<400> 1

Asn Tyr Pro Asp Cys Thr Thr Gly Pro Leu Ser Glu Leu Pro Ile Cys
1               5                   10                  15

Asp Thr Ser Leu Ser Pro Leu Glu Arg Ala Lys Ser Leu Val Ser Ala
            20                  25                  30

Leu Thr Leu Glu Glu Lys Ile Asn Asn Thr Gly His Glu Ala Ala Gly
        35                  40                  45

Ser Ser Arg Leu Gly Leu Pro Ala Tyr Asn Trp Trp Asn Glu Ala Leu
    50                  55                  60

His Gly Val Ala Glu Lys His Gly Val Ser Phe Glu Glu Ser Gly Asp
65                  70                  75                  80

Phe Ser Tyr Ala Thr Ser Phe Pro Ala Pro Ile Val Leu Gly Ala Ala
                85                  90                  95

Phe Asn Asp Ala Leu Ile Arg Arg Val Ala Glu Ile Ile Ser Thr Glu
            100                 105                 110

Ala Arg Ala Phe Ser Asn Ser Asp His Ala Gly Ile Asp Tyr Trp Thr
        115                 120                 125

Pro Asn Val Asn Pro Phe Lys Asp Pro Arg Trp Gly Arg Gly Gln Glu
    130                 135                 140

Thr Pro Gly Glu Asp Pro Leu His Cys Ser Arg Tyr Val Lys Glu Phe
145                 150                 155                 160

Val Gly Gly Leu Gln Gly Asp Asp Pro Glu Lys Pro Lys Val Val Ala
                165                 170                 175

Thr Cys Lys His Leu Ala Ala Tyr Asp Leu Glu Glu Trp Gly Gly Val
            180                 185                 190

Ser Arg Phe Glu Phe Asp Ala Lys Val Ser Ala Val Asp Leu Leu Glu
        195                 200                 205

Tyr Tyr Leu Pro Pro Phe Lys Thr Cys Ala Val Asp Ala Ser Val Gly
    210                 215                 220

Ala Phe Met Cys Ser Tyr Asn Ala Leu Asn Gly Val Pro Ala Cys Ala
225                 230                 235                 240

Asp Arg Tyr Leu Leu Gln Thr Val Leu Arg Glu His Trp Gly Trp Glu
                245                 250                 255

Gly Pro Gly His Trp Val Thr Gly Asp Cys Gly Ala Val Glu Arg Ile
            260                 265                 270

Gln Thr Tyr His His Tyr Val Glu Ser Gly Pro Glu Ala Ala Ala Ala
        275                 280                 285

Ala Leu Asn Ala Gly Val Asp Leu Asp Cys Gly Thr Trp Leu Pro Ser
    290                 295                 300

Tyr Leu Gly Glu Ala Glu Arg Gln Gly Leu Ile Ser Asn Glu Thr Leu
305                 310                 315                 320

Asp Ala Ala Leu Thr Arg Leu Tyr Thr Ser Leu Val Gln Leu Gly Tyr
                325                 330                 335

Phe Asp Pro Ala Glu Gly Gln Pro Leu Arg Ser Leu Gly Trp Asp Asp
            340                 345                 350

Val Ala Thr Ser Glu Ala Glu Glu Leu Ala Lys Thr Val Ala Ile Gln
        355                 360                 365

Gly Thr Val Leu Leu Lys Asn Ile Asp Trp Thr Leu Pro Leu Lys Ala
    370                 375                 380

Asn Gly Thr Leu Ala Leu Ile Gly Pro Phe Ile Asn Phe Thr Thr Glu
385                 390                 395                 400

Leu Gln Ser Asn Tyr Ala Gly Pro Ala Lys His Ile Pro Thr Met Ile
                405                 410                 415

Glu Ala Ala Glu Arg Leu Gly Tyr Asn Val Leu Thr Ala Pro Gly Thr
            420                 425                 430

Glu Val Asn Ser Thr Ser Thr Asp Gly Phe Asp Asp Ala Leu Ala Ile
        435                 440                 445

Ala Ala Glu Ala Asp Ala Leu Ile Phe Phe Gly Gly Ile Asp Asn Thr
    450                 455                 460

Val Glu Glu Glu Ser Leu Asp Arg Thr Arg Ile Asp Trp Pro Gly Asn
465                 470                 475                 480

Gln Glu Glu Leu Ile Leu Glu Leu Ala Glu Leu Gly Arg Pro Leu Thr
                485                 490                 495

Val Val Gln Phe Gly Gly Gly Gln Val Asp Asp Ser Ala Leu Leu Ala
            500                 505                 510

Ser Ala Gly Val Gly Ala Ile Val Trp Ala Gly Tyr Pro Ser Gln Ala
        515                 520                 525

Gly Gly Ala Gly Val Phe Asp Val Leu Thr Gly Lys Ala Ala Pro Ala
    530                 535                 540

Gly Arg Leu Pro Ile Thr Gln Tyr Pro Lys Ser Tyr Val Asp Glu Val
545                 550                 555                 560

Pro Met Thr Asp Met Asn Leu Gln Pro Gly Thr Asp Asn Pro Gly Arg
                565                 570                 575

Thr Tyr Arg Trp Tyr Glu Asp Ala Val Leu Pro Phe Gly Phe Gly Leu
            580                 585                 590

His Tyr Thr Thr Phe Asn Val Ser Trp Ala Lys Lys Ala Phe Gly Pro
        595                 600                 605

Tyr Asp Ala Ala Thr Leu Ala Arg Gly Lys Asn Pro Ser Ser Asn Ile
    610                 615                 620

Val Asp Thr Phe Ser Leu Ala Val Thr Asn Thr Gly Asp Val Ala Ser
625                 630                 635                 640

Asp Tyr Val Ala Leu Val Phe Ala Ser Ala Pro Glu Leu Gly Ala Gln
                645                 650                 655

Pro Ala Pro Ile Lys Thr Leu Val Gly Tyr Ser Arg Ala Ser Leu Ile
            660                 665                 670

Lys Pro Gly Glu Thr Arg Lys Val Asp Val Glu Val Thr Val Ala Pro
        675                 680                 685

Leu Thr Arg Ala Thr Glu Asp Gly Arg Val Val Leu Tyr Pro Gly Glu
    690                 695                 700

Tyr Thr Leu Leu Val Asp Val Asn Asp Glu Tyr Pro Thr Ala Lys Phe
705                 710                 715                 720

Glu Ile Lys Gly Asp Val Gln Val Leu Glu Lys Phe Pro Leu Ser Gly
                725                 730                 735

Asn Asp Ser Asp
            740

<210>  2
<211>  18
<212>  PRT
<213>  Aspergillus awamori

<400>  2

Met Ser Phe Arg Ser Leu Leu Ala Leu Ser Gly Leu Val Cys Ser Gly
1               5                   10                  15

Leu Ala

<210>  3
<211>  758
<212>  PRT
<213>  Artificial Sequence

<220>
<223>  fusion protein comprising the glucoamylase A signal peptide from
       Aspergillus awamori bound to the mature beta-xylosidase enzyme
       from Aspergillus nidulans.

<400>  3

Met Ser Phe Arg Ser Leu Leu Ala Leu Ser Gly Leu Val Cys Ser Gly
1               5                   10                  15

Leu Ala Asn Tyr Pro Asp Cys Thr Thr Gly Pro Leu Ser Glu Leu Pro
            20                  25                  30

Ile Cys Asp Thr Ser Leu Ser Pro Leu Glu Arg Ala Lys Ser Leu Val
        35                  40                  45

Ser Ala Leu Thr Leu Glu Glu Lys Ile Asn Asn Thr Gly His Glu Ala
    50                  55                  60

Ala Gly Ser Ser Arg Leu Gly Leu Pro Ala Tyr Asn Trp Trp Asn Glu
65                  70                  75                  80

Ala Leu His Gly Val Ala Glu Lys His Gly Val Ser Phe Glu Glu Ser
                85                  90                  95

Gly Asp Phe Ser Tyr Ala Thr Ser Phe Pro Ala Pro Ile Val Leu Gly
            100                 105                 110

Ala Ala Phe Asn Asp Ala Leu Ile Arg Arg Val Ala Glu Ile Ile Ser
        115                 120                 125

Thr Glu Ala Arg Ala Phe Ser Asn Ser Asp His Ala Gly Ile Asp Tyr
    130                 135                 140

Trp Thr Pro Asn Val Asn Pro Phe Lys Asp Pro Arg Trp Gly Arg Gly
145                 150                 155                 160

Gln Glu Thr Pro Gly Glu Asp Pro Leu His Cys Ser Arg Tyr Val Lys
                165                 170                 175

Glu Phe Val Gly Gly Leu Gln Gly Asp Asp Pro Glu Lys Pro Lys Val
            180                 185                 190

Val Ala Thr Cys Lys His Leu Ala Ala Tyr Asp Leu Glu Glu Trp Gly
        195                 200                 205

Gly Val Ser Arg Phe Glu Phe Asp Ala Lys Val Ser Ala Val Asp Leu
    210                 215                 220

Leu Glu Tyr Tyr Leu Pro Pro Phe Lys Thr Cys Ala Val Asp Ala Ser
225                 230                 235                 240

Val Gly Ala Phe Met Cys Ser Tyr Asn Ala Leu Asn Gly Val Pro Ala
                245                 250                 255

Cys Ala Asp Arg Tyr Leu Leu Gln Thr Val Leu Arg Glu His Trp Gly
            260                 265                 270

Trp Glu Gly Pro Gly His Trp Val Thr Gly Asp Cys Gly Ala Val Glu
        275                 280                 285

Arg Ile Gln Thr Tyr His His Tyr Val Glu Ser Gly Pro Glu Ala Ala
    290                 295                 300

Ala Ala Ala Leu Asn Ala Gly Val Asp Leu Asp Cys Gly Thr Trp Leu
305                 310                 315                 320

Pro Ser Tyr Leu Gly Glu Ala Glu Arg Gln Gly Leu Ile Ser Asn Glu
                325                 330                 335

Thr Leu Asp Ala Ala Leu Thr Arg Leu Tyr Thr Ser Leu Val Gln Leu
            340                 345                 350

Gly Tyr Phe Asp Pro Ala Glu Gly Gln Pro Leu Arg Ser Leu Gly Trp
        355                 360                 365

Asp Asp Val Ala Thr Ser Glu Ala Glu Glu Leu Ala Lys Thr Val Ala
    370                 375                 380

Ile Gln Gly Thr Val Leu Leu Lys Asn Ile Asp Trp Thr Leu Pro Leu
385                 390                 395                 400

Lys Ala Asn Gly Thr Leu Ala Leu Ile Gly Pro Phe Ile Asn Phe Thr
                405                 410                 415

Thr Glu Leu Gln Ser Asn Tyr Ala Gly Pro Ala Lys His Ile Pro Thr
            420                 425                 430

Met Ile Glu Ala Ala Glu Arg Leu Gly Tyr Asn Val Leu Thr Ala Pro
        435                 440                 445

Gly Thr Glu Val Asn Ser Thr Ser Thr Asp Gly Phe Asp Asp Ala Leu
    450                 455                 460

Ala Ile Ala Ala Glu Ala Asp Ala Leu Ile Phe Phe Gly Gly Ile Asp
465                 470                 475                 480

Asn Thr Val Glu Glu Glu Ser Leu Asp Arg Thr Arg Ile Asp Trp Pro
                485                 490                 495

Gly Asn Gln Glu Glu Leu Ile Leu Glu Leu Ala Glu Leu Gly Arg Pro
            500                 505                 510

Leu Thr Val Val Gln Phe Gly Gly Gly Gln Val Asp Asp Ser Ala Leu
        515                 520                 525

Leu Ala Ser Ala Gly Val Gly Ala Ile Val Trp Ala Gly Tyr Pro Ser
    530                 535                 540

Gln Ala Gly Gly Ala Gly Val Phe Asp Val Leu Thr Gly Lys Ala Ala
545                 550                 555                 560

Pro Ala Gly Arg Leu Pro Ile Thr Gln Tyr Pro Lys Ser Tyr Val Asp
                565                 570                 575

Glu Val Pro Met Thr Asp Met Asn Leu Gln Pro Gly Thr Asp Asn Pro
            580                 585                 590

Gly Arg Thr Tyr Arg Trp Tyr Glu Asp Ala Val Leu Pro Phe Gly Phe
        595                 600                 605

Gly Leu His Tyr Thr Thr Phe Asn Val Ser Trp Ala Lys Lys Ala Phe
    610                 615                 620

Gly Pro Tyr Asp Ala Ala Thr Leu Ala Arg Gly Lys Asn Pro Ser Ser
625                 630                 635                 640

Asn Ile Val Asp Thr Phe Ser Leu Ala Val Thr Asn Thr Gly Asp Val
                645                 650                 655

Ala Ser Asp Tyr Val Ala Leu Val Phe Ala Ser Ala Pro Glu Leu Gly
            660                 665                 670

Ala Gln Pro Ala Pro Ile Lys Thr Leu Val Gly Tyr Ser Arg Ala Ser
        675                 680                 685

Leu Ile Lys Pro Gly Glu Thr Arg Lys Val Asp Val Glu Val Thr Val
    690                 695                 700

Ala Pro Leu Thr Arg Ala Thr Glu Asp Gly Arg Val Val Leu Tyr Pro
705                 710                 715                 720

Gly Glu Tyr Thr Leu Leu Val Asp Val Asn Asp Glu Tyr Pro Thr Ala
                725                 730                 735

Lys Phe Glu Ile Lys Gly Asp Val Gln Val Leu Glu Lys Phe Pro Leu
            740                 745                 750

Ser Gly Asn Asp Ser Asp
        755

<210>  4
<211>  2223
<212>  DNA
<213>  Aspergillus nidulans

<400>  4
aactaccccg actgcaccac cggccccctc tccgagctgc ccatctgcga cacctccctc      60

tcccccctgg agcgcgccaa gtccctcgtc tccgccctca ccctggagga gaagatcaac     120

aacaccggcc acgaggccgc cggctcctcc cgcctcggcc tccccgccta caactggtgg     180

aacgaggccc tccacggcgt cgccgagaag cacggcgtct ccttcgagga gtccggcgac     240

ttctcctacg ccacctcctt ccccgccccc atcgtcctcg gcgccgcctt caacgacgcc     300

ctcatccgcc gcgtcgccga gatcatctcc accgaggccc gcgccttctc caactccgac     360

cacgccggca tcgactactg gacccccaac gtcaacccct tcaaggaccc ccgctggggc     420

cgcggccagg agacccccgg cgaggacccc ctccactgct cccgctacgt caaggagttc     480

gtcggcggcc tccagggcga cgaccccgag aagcccaagg tcgtcgccac ctgcaagcac     540

ctcgccgcct acgacctgga ggagtggggc ggcgtctccc gcttcgagtt cgacgccaag     600

gtctccgccg tggacctcct ggagtactac ctccccccct tcaagacctg cgccgtggac     660

gcctccgtcg gcgccttcat gtgctcctac aacgccctca acggcgtccc cgcctgcgcc     720

gaccgctacc tcctccagac cgtcctccgc gagcactggg gctgggaggg ccccggccac     780

tgggtcaccg gcgactgcgg cgccgtcgag cgcatccaga cctaccacca ctacgtcgag     840

tccggccccg aggccgccgc cgccgccctc aacgccggcg tggacctcga ctgcggcacc     900

tggctcccct cctacctcgg cgaggccgag cgccagggcc tcatctccaa cgagaccctc     960

gacgccgccc tcacccgcct ctacacctcc ctcgtccagc tcggctactt cgaccccgcc    1020

gagggccagc ccctccgctc cctcggctgg gacgacgtcg ccacctccga ggccgaggag    1080

ctggccaaga ccgtcgccat ccagggcacc gtcctcctca agaacatcga ctggaccctc    1140

cccctcaagg ccaacggcac cctcgccctc atcggcccct tcatcaactt caccaccgag    1200

ctgcagtcca actacgccgg ccccgccaag cacatcccca ccatgatcga ggccgccgag    1260

cgcctcggct acaacgtcct caccgccccc ggcaccgagg tcaactccac ctccaccgac    1320

ggcttcgacg acgccctcgc catcgccgcc gaggccgacg ccctcatctt cttcggcggc    1380

atcgacaaca ccgtcgagga ggagtccctc gaccgcaccc gcatcgactg gcccggcaac    1440

caggaggagc tgatcctgga gctggccgag ctgggccgcc ccctcaccgt cgtccagttc    1500

ggcggcggcc aggtggacga ctccgccctc ctcgcctccg ccggcgtcgg cgccatcgtc    1560

tgggccggct acccctccca ggccggcggc gccggcgtct tcgacgtcct caccggcaag 1620

gccgcccccg ccggccgcct ccccatcacc cagtacccca agtcctacgt ggacgaggtc 1680

cccatgaccg acatgaacct ccagcccggc accgacaacc ccggccgcac ctaccgctgg 1740

tacgaggacg ccgtcctccc cttcggcttc ggcctccact acaccacctt caacgtctcc 1800

tgggccaaga aggcgttcgg cccctacgac gccgccaccc tcgcccgcgg caagaacccc 1860

tcctccaaca tcgtggacac cttctccctc gccgtcacca acaccggcga cgtcgcctcc 1920

gactacgtcg ccctcgtctt cgcctccgcc cccgagctgg gcgcccagcc cgcccccatc 1980

aagaccctcg tcggctactc ccgcgcctcc ctcatcaagc ccggcgagac ccgcaaggtg 2040

gacgtcgagg tcaccgtcgc ccccctcacc cgcgccaccg aggacggccg cgtcgtcctc 2100

taccccggcg agtacaccct cctcgtggac gtcaacgacg agtaccccac cgccaagttc 2160

gagatcaagg gcgacgtcca ggtcctggag aagttccccc tctccggcaa cgactccgac 2220

taa 2223

<210> 5
<211> 54
<212> DNA
<213> Aspergillus awamori

<400> 5
atgtcgttcc gatctcttct cgccctgagc ggccttgtct gctcggggtt ggca 54

<210> 6
<211> 2277
<212> DNA
<213> Artificial sequence

<220>
<223> Nucleotide sequence encoding the fusion protein SEQ ID NO: 3

<400> 6
atgtcgttcc gatctcttct cgccctgagc ggccttgtct gctcggggtt ggcaaactac 60

cccgactgca ccaccggccc cctctccgag ctgcccatct gcgacacctc cctctccccc 120

ctggagcgcg ccaagtccct cgtctccgcc ctcaccctgg aggagaagat caacaacacc 180

ggccacgagg ccgccggctc ctcccgcctc ggcctccccg cctacaactg gtggaacgag 240

gccctccacg gcgtcgccga gaagcacggc gtctccttcg aggagtccgg cgacttctcc 300

tacgccacct ccttccccgc ccccatcgtc ctcggcgccg ccttcaacga cgccctcatc 360

cgccgcgtcg ccgagatcat ctccaccgag gcccgcgcct tctccaactc cgaccacgcc 420

ggcatcgact actggacccc caacgtcaac cccttcaagg acccccgctg gggccgcggc 480

caggagaccc ccggcgagga ccccctccac tgctcccgct acgtcaagga gttcgtcggc 540

ggcctccagg gcgacgaccc cgagaagccc aaggtcgtcg ccacctgcaa gcacctcgcc 600

gcctacgacc tggaggagtg gggcggcgtc tcccgcttcg agttcgacgc caaggtctcc 660

gccgtggacc tcctggagta ctacctcccc cccttcaaga cctgcgccgt ggacgcctcc 720

gtcggcgcct tcatgtgctc ctacaacgcc ctcaacggcg tccccgcctg cgccgaccgc 780

tacctcctcc agaccgtcct ccgcgagcac tggggctggg agggccccgg ccactgggtc 840
accggcgact gcggcgccgt cgagcgcatc cagacctacc accactacgt cgagtccggc 900
cccgaggccg ccgccgccgc cctcaacgcc ggcgtggacc tcgactgcgg cacctggctc 960
ccctcctacc tcggcgaggc cgagcgccag ggcctcatct ccaacgagac cctcgacgcc 1020
gccctcaccc gcctctacac ctccctcgtc cagctcggct acttcgaccc cgccgagggc 1080
cagcccctcc gctccctcgg ctgggacgac gtcgccacct ccgaggccga ggagctggcc 1140
aagaccgtcg ccatccaggg caccgtcctc ctcaagaaca tcgactggac cctccccctc 1200
aaggccaacg gcaccctcgc cctcatcggc cccttcatca acttcaccac cgagctgcag 1260
tccaactacg ccggccccgc caagcacatc cccaccatga tcgaggccgc cgagcgcctc 1320
ggctacaacg tcctcaccgc ccccggcacc gaggtcaact ccacctccac cgacggcttc 1380
gacgacgccc tcgccatcgc cgccgaggcc gacgccctca tcttcttcgg cggcatcgac 1440
aacaccgtcg aggaggagtc cctcgaccgc acccgcatcg actggcccgg caaccaggag 1500
gagctgatcc tggagctggc cgagctgggc cgccccctca ccgtcgtcca gttcggcggc 1560
ggccaggtgg acgactccgc cctcctcgcc tccgccggcg tcggcgccat cgtctgggcc 1620
ggctacccct cccaggccgg cggcgccggc gtcttcgacg tcctcaccgg caaggccgcc 1680
cccgccggcc gcctccccat cacccagtac cccaagtcct acgtggacga ggtccccatg 1740
accgacatga acctccagcc cggcaccgac aaccccggcc gcacctaccg ctggtacgag 1800
gacgccgtcc tccccttcgg cttcggcctc cactacacca ccttcaacgt ctcctgggcc 1860
aagaaggcgt tcggccccta cgacgccgcc accctcgccc gcggcaagaa cccctcctcc 1920
aacatcgtgg acaccttctc cctcgccgtc accaacaccg gcgacgtcgc ctccgactac 1980
gtcgccctcg tcttcgcctc cgcccccgag ctgggcgccc agcccgcccc catcaagacc 2040
ctcgtcggct actcccgcgc ctccctcatc aagcccggcg agacccgcaa ggtggacgtc 2100
gaggtcaccg tcgcccccct cacccgcgcc accgaggacg gccgcgtcgt cctctacccc 2160
ggcgagtaca ccctcctcgt ggacgtcaac gacgagtacc ccaccgccaa gttcgagatc 2220
aagggcgacg tccaggtcct ggagaagttc cccctctccg gcaacgactc cgactaa 2277

<210> 7
<211> 763
<212> PRT
<213> Aspergillus nidulans


<220>
<221> SIGNAL
<222> (1)..(23)
<223> Signal peptide

<400> 7

Met Ala Val Phe Lys Ser Trp Asn Leu Ala Leu Leu Ser Ser Leu Phe
1               5                   10                  15

Ile Pro Ala Leu Cys Gln Ser Asn Tyr Pro Asp Cys Thr Thr Gly Pro
            20                  25                  30
Leu Ser Glu Leu Pro Ile Cys Asp Thr Ser Leu Ser Pro Leu Glu Arg
        35                  40                  45
Ala Lys Ser Leu Val Ser Ala Leu Thr Leu Glu Glu Lys Ile Asn Asn
    50                  55                  60
Thr Gly His Glu Ala Ala Gly Ser Ser Arg Leu Gly Leu Pro Ala Tyr
65                  70                  75                  80
Asn Trp Trp Asn Glu Ala Leu His Gly Val Ala Glu Lys His Gly Val
                85                  90                  95
Ser Phe Glu Glu Ser Gly Asp Phe Ser Tyr Ala Thr Ser Phe Pro Ala
            100                 105                 110
Pro Ile Val Leu Gly Ala Ala Phe Asn Asp Ala Leu Ile Arg Arg Val
        115                 120                 125
Ala Glu Ile Ile Ser Thr Glu Ala Arg Ala Phe Ser Asn Ser Asp His
    130                 135                 140
Ala Gly Ile Asp Tyr Trp Thr Pro Asn Val Asn Pro Phe Lys Asp Pro
145                 150                 155                 160
Arg Trp Gly Arg Gly Gln Glu Thr Pro Gly Glu Asp Pro Leu His Cys
                165                 170                 175
Ser Arg Tyr Val Lys Glu Phe Val Gly Gly Leu Gln Gly Asp Asp Pro
            180                 185                 190
Glu Lys Pro Lys Val Val Ala Thr Cys Lys His Leu Ala Ala Tyr Asp
        195                 200                 205
Leu Glu Glu Trp Gly Gly Val Ser Arg Phe Glu Phe Asp Ala Lys Val
    210                 215                 220
Ser Ala Val Asp Leu Leu Glu Tyr Tyr Leu Pro Pro Phe Lys Thr Cys
225                 230                 235                 240
Ala Val Asp Ala Ser Val Gly Ala Phe Met Cys Ser Tyr Asn Ala Leu
                245                 250                 255
Asn Gly Val Pro Ala Cys Ala Asp Arg Tyr Leu Leu Gln Thr Val Leu
            260                 265                 270
Arg Glu His Trp Gly Trp Glu Gly Pro Gly His Trp Val Thr Gly Asp
        275                 280                 285
Cys Gly Ala Val Glu Arg Ile Gln Thr Tyr His His Tyr Val Glu Ser

    290                 295                 300

Gly Pro Glu Ala Ala Ala Ala Ala Leu Asn Ala Gly Val Asp Leu Asp
305                 310                 315                 320

Cys Gly Thr Trp Leu Pro Ser Tyr Leu Gly Glu Ala Glu Arg Gln Gly
                325                 330                 335

Leu Ile Ser Asn Glu Thr Leu Asp Ala Ala Leu Thr Arg Leu Tyr Thr
            340                 345                 350

Ser Leu Val Gln Leu Gly Tyr Phe Asp Pro Ala Glu Gly Gln Pro Leu
        355                 360                 365

Arg Ser Leu Gly Trp Asp Asp Val Ala Thr Ser Glu Ala Glu Glu Leu
    370                 375                 380

Ala Lys Thr Val Ala Ile Gln Gly Thr Val Leu Leu Lys Asn Ile Asp
385                 390                 395                 400

Trp Thr Leu Pro Leu Lys Ala Asn Gly Thr Leu Ala Leu Ile Gly Pro
                405                 410                 415

Phe Ile Asn Phe Thr Thr Glu Leu Gln Ser Asn Tyr Ala Gly Pro Ala
            420                 425                 430

Lys His Ile Pro Thr Met Ile Glu Ala Ala Glu Arg Leu Gly Tyr Asn
        435                 440                 445

Val Leu Thr Ala Pro Gly Thr Glu Val Asn Ser Thr Ser Thr Asp Gly
    450                 455                 460

Phe Asp Asp Ala Leu Ala Ile Ala Ala Glu Ala Asp Ala Leu Ile Phe
465                 470                 475                 480

Phe Gly Gly Ile Asp Asn Thr Val Glu Glu Glu Ser Leu Asp Arg Thr
                485                 490                 495

Arg Ile Asp Trp Pro Gly Asn Gln Glu Glu Leu Ile Leu Glu Leu Ala
            500                 505                 510

Glu Leu Gly Arg Pro Leu Thr Val Val Gln Phe Gly Gly Gly Gln Val
        515                 520                 525

Asp Asp Ser Ala Leu Leu Ala Ser Ala Gly Val Gly Ala Ile Val Trp
    530                 535                 540

Ala Gly Tyr Pro Ser Gln Ala Gly Gly Ala Gly Val Phe Asp Val Leu
545                 550                 555                 560

Thr Gly Lys Ala Ala Pro Ala Gly Arg Leu Pro Ile Thr Gln Tyr Pro
                565                 570                 575

Lys Ser Tyr Val Asp Glu Val Pro Met Thr Asp Met Asn Leu Gln Pro
            580                 585                 590

Gly Thr Asp Asn Pro Gly Arg Thr Tyr Arg Trp Tyr Glu Asp Ala Val
        595                 600                 605

Leu Pro Phe Gly Phe Gly Leu His Tyr Thr Thr Phe Asn Val Ser Trp
    610                 615                 620

Ala Lys Lys Ala Phe Gly Pro Tyr Asp Ala Ala Thr Leu Ala Arg Gly
625                 630                 635                 640

Lys Asn Pro Ser Ser Asn Ile Val Asp Thr Phe Ser Leu Ala Val Thr
                645                 650                 655

Asn Thr Gly Asp Val Ala Ser Asp Tyr Val Ala Leu Val Phe Ala Ser
            660                 665                 670

Ala Pro Glu Leu Gly Ala Gln Pro Ala Pro Ile Lys Thr Leu Val Gly
        675                 680                 685

Tyr Ser Arg Ala Ser Leu Ile Lys Pro Gly Glu Thr Arg Lys Val Asp
    690                 695                 700

Val Glu Val Thr Val Ala Pro Leu Thr Arg Ala Thr Glu Asp Gly Arg
705                 710                 715                 720

Val Val Leu Tyr Pro Gly Glu Tyr Thr Leu Leu Val Asp Val Asn Asp
                725                 730                 735

Glu Tyr Pro Thr Ala Lys Phe Glu Ile Lys Gly Asp Val Gln Val Leu
            740                 745                 750

Glu Lys Phe Pro Leu Ser Gly Asn Asp Ser Asp
        755                 760

<210> 8
<211> 327
<212> PRT
<213> Humicola insolens

<400> 8

Met Ala Pro Leu Ile Thr Asn Ile Tyr Thr Ala Asp Pro Ser Ala His
1               5                   10                  15

Val Phe Asn Gly Lys Leu Tyr Ile Tyr Pro Ser His Asp Arg Glu Thr
            20                  25                  30

Asp Ile Gln Phe Asn Asp Asn Gly Asp Gln Tyr Asp Met Ala Asp Tyr
        35                  40                  45

His Val Phe Ser Leu Asp Ser Leu Asp Pro Pro Ser Glu Val Thr Asp
    50                  55                  60

His Gly Val Val Leu Lys Val Glu Asp Ile Pro Trp Val Ser Lys Gln
65                  70                  75                  80

Leu Trp Ala Pro Asp Ala Ala Thr Lys Asp Gly Lys Tyr Tyr Leu Tyr
                85                  90                  95

Phe Pro Ala Arg Asp Lys Glu Gly Ile Phe Arg Ile Gly Val Ala Val
            100                 105                 110

Ser Asp Lys Pro Glu Gly Pro Phe Thr Pro Asp Pro Glu Pro Ile Lys
        115                 120                 125

Gly Ser Tyr Ser Ile Asp Pro Ala Val Phe Val Asp Asp Asp Gly Ser
    130                 135                 140

Ala Tyr Met Tyr Phe Gly Gly Leu Trp Gly Gly Gln Leu Gln Cys Tyr
145                 150                 155                 160

Gln Lys Gly Asn Asn Ile Phe Asp Ala Glu Trp Ser Gly Pro Lys Glu
                165                 170                 175

Pro Ser Gly Ser Gly Ala Lys Ala Leu Gly Pro Arg Val Ala Lys Leu
            180                 185                 190

Thr Asp Asp Met Arg Gln Phe Ala Glu Glu Val Arg Glu Ile Val Ile
        195                 200                 205

Leu Ala Pro Glu Thr Gly Glu Pro Leu Ala Ala Asp Asp His Asp Arg
    210                 215                 220

Arg Phe Phe Glu Ala Ala Trp Met His Lys Tyr Asn Gly Lys Tyr Tyr
225                 230                 235                 240

Phe Ser Tyr Ser Thr Gly Asp Thr His Tyr Leu Val Tyr Ala Val Gly
                245                 250                 255

Asp Ser Pro Tyr Gly Pro Phe Thr Tyr Gly Gly Arg Ile Leu Glu Pro
            260                 265                 270

Val Leu Gly Trp Thr Thr His His Ser Ile Val Glu Phe Gln Gly Arg
        275                 280                 285

Trp Trp Leu Phe His His Asp Cys Glu Leu Ser Lys Gly Val Asp His
    290                 295                 300

Leu Arg Ser Val Lys Val Lys Glu Ile Trp Tyr Asp Lys Asp Gly Lys
305                 310                 315                 320

Ile Val Thr Glu Lys Pro Glu

                325

<210> 9
<211> 981
<212> DNA
<213> Humicola insolens

<400> 9
gcccccctca tcaccaacat ctacaccgcc gacccctccg cccacgtctt caacggcaag 60

ctctacatct acccctccca cgaccgcgag accgacatcc agttcaacga caacggcgac 120

cagtacgaca tggccgacta ccacgtcttc tccctcgact ccctcgaccc cccctccgag 180

gtcaccgacc acggcgtcgt cctcaaggtc gaggacatcc cctgggtctc caagcagctc 240

tgggcccccg acgccgccac caaggacggc aagtactacc tctacttccc cgcccgcgac 300

aaggagggca tcttccgcat cggcgtcgcc gtctccgaca agcccgaggg ccccttcacc 360

cccgaccccg agcccatcaa gggctcctac tccatcgacc ccgccgtctt cgtcgacgac 420

gacggctccg cctacatgta cttcggcggc ctctggggcg gccagctcca gtgctaccag 480

aagggcaaca acatcttcga cgccgagtgg tccggcccca aggagccctc cggctccggc 540

gccaaggccc tcggcccccg cgtcgccaag ctcaccgacg acatgcgcca gttcgccgag 600

gaggtccgcg agatcgtcat cctcgccccc gagaccggcg agcccctcgc cgccgacgac 660

cacgaccgcc gcttcttcga ggccgcctgg atgcacaagt acaacggcaa gtactacttc 720

tcctactcca ccggcgacac ccactacctc gtctacgccg tcggcgactc cccctacggc 780

cccttcacct acggcggccg catcctcgag cccgtcctcg gctggaccac ccaccactcc 840

atcgtcgagt tccagggccg ctggtggctc ttccaccacg actgcgagct ctccaagggc 900

gtcgaccacc tccgctccgt caaggtcaag gagatctggt acgacaagga cggcaagatc 960

gtcaccgaga agcccgagta a 981

<210> 10
<211> 344
<212> PRT
<213> Artificial sequence

<220>
<223> Fusion protein comprising the glucoamylase A signal peptide from
Aspergillus awamori bound to the beta-xylosidase enzyme from
Humicola insolens

<400> 10

Met Ser Phe Arg Ser Leu Leu Ala Leu Ser Gly Leu Val Cys Ser Gly
1               5                   10                  15

Leu Ala Ala Pro Leu Ile Thr Asn Ile Tyr Thr Ala Asp Pro Ser Ala
            20                  25                  30

His Val Phe Asn Gly Lys Leu Tyr Ile Tyr Pro Ser His Asp Arg Glu
        35                  40                  45

Thr Asp Ile Gln Phe Asn Asp Asn Gly Asp Gln Tyr Asp Met Ala Asp

    50                  55                  60

Tyr His Val Phe Ser Leu Asp Ser Leu Asp Pro Pro Ser Glu Val Thr
65                  70                  75                  80

Asp His Gly Val Val Leu Lys Val Glu Asp Ile Pro Trp Val Ser Lys
                85                  90                  95

Gln Leu Trp Ala Pro Asp Ala Ala Thr Lys Asp Gly Lys Tyr Tyr Leu
            100                 105                 110

Tyr Phe Pro Ala Arg Asp Lys Glu Gly Ile Phe Arg Ile Gly Val Ala
        115                 120                 125

Val Ser Asp Lys Pro Glu Gly Pro Phe Thr Pro Asp Pro Glu Pro Ile
    130                 135                 140

Lys Gly Ser Tyr Ser Ile Asp Pro Ala Val Phe Val Asp Asp Asp Gly
145                 150                 155                 160

Ser Ala Tyr Met Tyr Phe Gly Gly Leu Trp Gly Gly Gln Leu Gln Cys
                165                 170                 175

Tyr Gln Lys Gly Asn Asn Ile Phe Asp Ala Glu Trp Ser Gly Pro Lys
            180                 185                 190

Glu Pro Ser Gly Ser Gly Ala Lys Ala Leu Gly Pro Arg Val Ala Lys
        195                 200                 205

Leu Thr Asp Asp Met Arg Gln Phe Ala Glu Glu Val Arg Glu Ile Val
    210                 215                 220

Ile Leu Ala Pro Glu Thr Gly Glu Pro Leu Ala Ala Asp Asp His Asp
225                 230                 235                 240

Arg Arg Phe Phe Glu Ala Ala Trp Met His Lys Tyr Asn Gly Lys Tyr
                245                 250                 255

Tyr Phe Ser Tyr Ser Thr Gly Asp Thr His Tyr Leu Val Tyr Ala Val
            260                 265                 270

Gly Asp Ser Pro Tyr Gly Pro Phe Thr Tyr Gly Gly Arg Ile Leu Glu
        275                 280                 285

Pro Val Leu Gly Trp Thr Thr His His Ser Ile Val Glu Phe Gln Gly
    290                 295                 300

Arg Trp Trp Leu Phe His His Asp Cys Glu Leu Ser Lys Gly Val Asp
305                 310                 315                 320

His Leu Arg Ser Val Lys Val Lys Glu Ile Trp Tyr Asp Lys Asp Gly
                325                 330                 335

Lys Ile Val Thr Glu Lys Pro Glu
            340

<210> 11
<211> 1035
<212> DNA
<213> Artificial sequence

<220>
<223> Nucleotide sequence encoding SEQ ID NO: 10

<400> 11
atgtcgttcc gatctcttct cgccctgagc ggccttgtct gctcggggtt ggcagccccc 60

ctcatcacca acatctacac cgccgacccc tccgcccacg tcttcaacgg caagctctac 120

atctacccct cccacgaccg cgagaccgac atccagttca acgacaacgg cgaccagtac 180

gacatggccg actaccacgt cttctccctc gactccctcg accccccctc cgaggtcacc 240

gaccacggcg tcgtcctcaa ggtcgaggac atcccctggg tctccaagca gctctgggcc 300

cccgacgccg ccaccaagga cggcaagtac tacctctact tccccgcccg cgacaaggag 360

ggcatcttcc gcatcggcgt cgccgtctcc gacaagcccg agggcccctt cacccccgac 420

cccgagccca tcaagggctc ctactccatc gaccccgccg tcttcgtgga cgacgacggc 480

tccgcctaca tgtacttcgg cggcctctgg ggcggccagc tccagtgcta ccagaagggc 540

aacaacatct tcgacgccga gtggtccggc cccaaggagc cctccggctc cggcgccaag 600

gccctcggcc cccgcgtcgc caagctcacc gacgacatgc gccagttcgc cgaggaggtc 660

cgcgagatcg tcatcctcgc ccccgagacc ggcgagcccc tcgccgccga cgaccacgac 720

cgccgcttct tcgaggccgc ctggatgcac aagtacaacg gcaagtacta cttctcctac 780

tccaccggcg acacccacta cctcgtctac gccgtcggcg actcccccta cggccccttc 840

acctacggcg ggcgcatcct ggagcccgtc ctcggctgga ccacccacca ctccatcgtc 900

gagttccagg gccgctggtg gctcttccac cacgactgcg agctgtccaa gggcgtggac 960

cacctccgct ccgtcaaggt caaggagatc tggtacgaca aggacggcaa gatcgtcacc 1020

gagaagcccg agtaa 1035

<210> 12
<211> 18
<212> PRT
<213> Aspergillus niger

<400> 12

Met Ser Phe Arg Ser Leu Leu Ala Leu Ser Gly Leu Val Cys Thr Gly
1               5                   10                  15

Leu Ala

## Description

The invention relates to the field of bioproducts, preferably biofuels, and more particularly, to the expression of a recombinant enzyme with beta-xylosidase activity in host cells and its use in the production of bioproducts, preferably biofuels, from biomass.

### BACKGROUND ART

Nowadays, many efforts are being made in order to obtain less expensive and renewable sources of fuel. Biofuels offer an attractive alternative to petroleum based fuels and can be obtained through the fermentation of monomeric sugars derived from starch or cellulose. However, current economics do not support the widespread use of biofuels due to the high cost of generating them.

Plant biomass provides a plentiful source of potential energy in form of carbohydrates that can be utilized for numerous industrial and agricultural processes, and is therefore a significant renewable resource for generating fermentable sugars. Fermentation of these sugars can commercially produce valuable end-products, such as biofuel and biochemicals. However, the enormous potential energy of these carbohydrates is currently under-utilized because the sugars are locked in complex polymers and, hence, are not readily accessible for fermentation (WO2012018691A2).

Wood, agricultural residues, herbaceous crops, and municipal solid wastes have been considered as feedstocks for ethanol production. These materials primarily consist of cellulose, hemicellulose, and/or lignin. Once the cellulose is converted to glucose by means of an enzymatic hydrolytic process, the glucose is easily fermented by yeast into ethanol. Thus, the more amounts of complex sugars remaining at the end of the hydrolytic process the lower the yield of ethanol production at the end of the fermentation process. Therefore, one area of research aimed at decreasing costs and enhancing the yield of biofuel production processes is focus on the enhancement of the technical efficacy of the hydrolytic enzymes that can be used to generate fermentable sugars from biomass.

Due to the complexity of biomass, its conversion to monomer sugars involves the action of several different enzyme classes, which digest cellulose and hemicellulose, major polysaccharides comprised in cellulosic materials. After cellulose, hemicellulose is the second most abundant fraction available in nature. It is a storage polymer in seeds and it forms the structural component in cell walls of woody plants. The classification of these hemicellulose fractions depends on the types of sugar moieties present. The principal monomers present in most of the hemicelluloses are D-xylose, D-mannose, D-galactose and L-arabinose. Thus, hemicellulose includes xylan, mannan, galactan and arabinan as the main heteropolymers. Specifically, xylan contains 85 to 93% of D-xylose, a small amount of arabinose and traces of glucuronic acid residues. The main chain of xylan is composed of beta-(1-4) linked beta-xylopyranose residues, and several side chains have been described to be present. Among them, most usually found are xylopiranose, glucuronic acid and arabinofuranose linkages, as well as acetyl groups (Bastawde, 1992, World Journal of Microbiology and Biotechnology (8) 353-368).

The presence of lignin in biomass leads to a protective barrier that prevents proper enzymatic hydrolysis of glucan and xylan. Thus, a pretreatment process of the biomass is required for increasing the access of the enzymes to their substrates and consequent efficient hydrolysis. Pretreatment uses various techniques, including ammonia fiber explosion, chemical treatment and steam explosion at high temperatures to alter the structure of cellulosic biomass and make cellulose more accessible. Hemicellulose can be readily hydrolysed under moderate conditions, but much more extreme conditions are needed for cellulose hydrolysis. Therefore, the pretreated material (substrate for the enzymatic hydrolysis) usually contains a high concentration of xylose, whereas glucose content is rather low (Kumar et al, 2009. Ind. Eng. Chem. Res., 48 (8), 3713-3729).

Single component enzymes have been shown to only partially digest cellulose and hemicellulose and thus the concerted action of different classes of enzymes is required to complete their conversion to monomeric sugars. Many more enzymes are required to digest hemicellulose to sugar monomers including xylanase, xylosidase, arabinofuranosidase, mannanase, galactosidase and glucuronidase.

Non-glycosyl hydrolases such as acetyl xylan esterase and ferulic acid esterase may also be involved.

A large number of naturally-occurring organisms have been found to produce enzymatic hydrolysis of cellulosic materials to produce fermentable sugars. Organisms capable of carry out a complete cellulose and hemicellulose degradation, that subsequently allows an efficient fermentation, would greatly enhance the cost effectiveness of bioethanol production.

The hydrolytic efficiency of a multi-enzyme complex in the process of cellulosic saccharification (or hydrolysis) depend both on properties of the individual enzymes and the ratio of each enzyme within the complex. It is therefore desirable to generate cellulolytic enzymes expressing-microorganisms which improve the yield of cellulosic material degradation process, increasing the amount of released fermentable sugars and thus improving the yield of final biofuel production.

Thus, some efforts carried out in order to generate improved cellulolytic enzymes expressing-microorganisms have involved inserting a gene encoding the specific hydrolytic enzyme to be expressed under the control of strong expression signals, which leads to an increased stability of the transcribed mRNA or an increased number of copies of the gene in the produced organism (US20080194005A1).

A number of host cells used for heterologous gene expression, such as bacteria *Escherichia coli,* and methods of transformation have been disclosed in the prior art. In this context, also a number of fungal expression systems have been developed, for instance *Aspergillus niger, Aspergillus awamori, Aspergillus nidulans, Trichoderma reesei.* However, for various reasons many of these recombinant microorganisms have not found wide-spread acceptance or use. In general terms, the ideal host cell must fulfill a large number of criteria, such as, uses the medium efficiently, produces the polypeptide or protein of interest in high yield, should be capable of efficient secretion of the protein or polypeptide, allows a wide range of expression regulatory elements to be used thus ensuring ease of application and versatility, allows the use of easily selectable markers that are cheap to use, and produce stable transformants.

### DESCRIPTION OF THE INVENTION

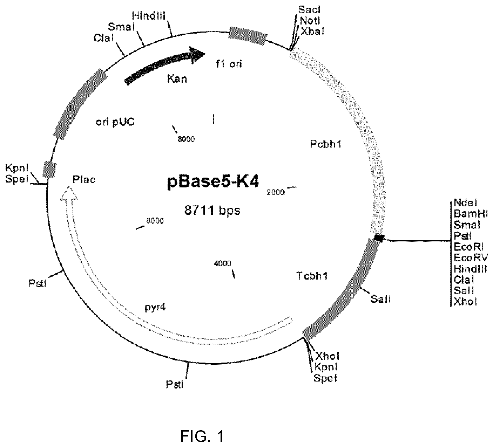

The present invention relates to the recombinant expression of a beta-xylosidase enzyme which comprises an amino acid sequence that is at least 70% identical to SEQ ID NO: 1, preferably the beta-xylosidase enzyme of SEQ ID NO: 1, in a host cell, more preferably in *Myceliophthora thermophila,* even more preferably in *Myceliophthora thermophila* strain C1. Said recombinant expression leads to a cell with improved efficiency of hydrolysis of biomass into fermentable sugars, more particularly the degradation of xylan oligomers to xylose (see figure 1), as compared with the wild type cell that does not express said recombinant beta-xylosidase or with another beta-xyosidase enzyme coming from a fungus different from A. *nidulans,* being thus useful in methods of producing bioproducts, preferably biofuel, from biomass.

The present invention represents a solution to the need to provide a microorganism that expresses a mixture of cellulolytic enzymes which improves the yield of biomass hydrolytic process or saccharification, increasing the amount of released fermentable sugars and thus improving the yield of bioproducts, preferably biofuel, obtained after the fermentative process.

An important percentage of xylose of constituent biomass polysaccharides is not released in the process of enzymatic hydrolysis of biomass. The host cell of the invention expresses a recombinant beta-xylosidase enzyme which is capable of degrading xylan oligomers to xylose in a more efficient way than other beta-xylosidase enzymes. Thus, this host cell and the enzymatic cocktail produced by it are useful for the optimization of the hydrolysis step of biomass into fermentable sugars.

The inventors have demonstrated that the incorporation, and successful later expression, of a recombinant beta-xylosidase enzyme which comprises an amino acid sequence having at least 70% sequence identity to SEQ ID NO: 1, preferably the mature beta-xylosidase AnBxl from the fungus *Aspergillus nidulans* which comprises the amino acid sequence SEQ ID NO: 1, in a *Myceliophthora thermophila* host cell, enhances the concentration of released xylose from biomass when the transformed cell or the enzymatic cocktail produced by said cell is used in a process of hydrolysis of biomass. This represents an increase in the final concentration of fermentable sugars, and hence of the overall yield of bioproducts, preferably biofuel, production.

Therefore, a first aspect of the present invention is related to a *Myceliophthora thermophila* host cell which expresses a nucleotide sequence encoding a recombinant beta-xylosidase enzyme comprising an amino acid sequence having at least 70% sequence identity to SEQ ID NO: 1, from now on referred to as "host cell of the invention".

The "host cell", as used herein, includes any cell type which is susceptible to transformation, transfection, transduction, and the like with a nucleic acid construct or expression vector comprising a nucleotide sequence or polynucleotide encoding the recombinant beta-xylosidase enzyme referred to above. The choice of a host cell will to a large extend depend upon the nucleotide sequence encoding the polypeptide and its source. The host cell may be eukaryote, such as mammalian, insect, plant or fungal cell. In a preferred embodiment, the host cell is a filamentous fungal cell. Filamentous fungi are generally characterized by a mycelial wall composed of chitin, cellulose, glucan, chitosan, mannan, and other complex polysaccharides. In a more preferred embodiment, the filamentous fungal host cell is an *Acremonium, Aspergillus, Aureobasidium, Bjerkandera, Ceriporiopsis, Coprinus, Coriolus, Cryptococcus, Filibasidium, Fusarium, Gibberella, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. In a most preferred embodiment, the filamentous fungal host cell is an *Aspergillus awamori, Aspergillus fumigatus, Aspergillus foetidus, Aspergillus japonicus, Aspergillus nidulans, Aspergillus niger* or *Aspergillus oryzae* cell. In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium pseudograminearum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another most preferred embodiment, the filamentous fungal host cell is a *Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Gibberella zeae, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell. In an even more preferred embodiment, the host cell of the invention is any strain of the species *Myceliophthora thermophila.* In a preferred embodiment, the host cell of the invention is *Myceliophthora thermophila* strain C1.

It will be understood that for the aforementioned species the invention encompasses both perfect and imperfect states, and other taxonomic equivalents, e. g. anamorphs, regardless of the species name by which they are known. Those skilled in the art will readily recognize the identity of appropriate equivalents. For instance, *Myceliophthora thermophila* is equivalent to *Chrysosporium lucknowense.*

The host cell of the invention expresses a nucleotide sequence encoding a recombinant beta-xylosidase enzyme comprising an amino acid sequence having at least 70% sequence identity to SEQ ID NO: 1.

The term "beta-xylosidase" refers to a protein that hydrolyses short 1,4-beta-D-xylooligomers into xylose. The "recombinant beta-xylosidase" of the invention is a beta-xylosidase enzyme which is naturally expressed in a microorganism other than the host cell of the invention, i.e. a heterologous beta-xylosidase, the amino acid sequence of which has not been modified or has been modified preferably by means of one or more deletions, insertions, substitutions, etc. In the context of the present invention, the terms "foreign" and "heterologous" are equivalent and mean derived from a different cell type or a different species from the recipient.

In a preferred embodiment, the recombinant beta-xylosidase is a naturally occurring beta-xylosidase coming from or isolated from a microorganism other than the host cell of the invention, more preferably from an *Aspergillus* cell, even more preferably from an *Aspergillus nidulans* cell.

The recombinant beta-xylosidase referred to in the present invention comprises an amino acid sequence having at least 70% sequence identity to SEQ ID NO: 1.
SEQ ID NO: 1:

NYPDCTTGPLSELPICDTSLSPLERAKSLVSALTLEEKINNTGHEAAGSSRLGLPAYNWW
NEALHGVAEKHGVSFEESGDFSYATSFPAPIVLGAAFNDALIRRVAEIISTEARAFSNSD
HAGIDYWTPNVNPFKDPRWGRGQETPGEDPLHCSRYVKEFVGGLQGDDPEKPKVVATCKH
LAAYDLEEWGGVSRFEFDAKVSAVDLLEYYLPPFKTCAVDASVGAFMCSYNALNGVPACA
DRYLLQTVLREHWGWEGPGHWVTGDCGAVERIQTYHHYVESGPEAAAAALNAGVDLDCGT
WLPSYLGEAERQGLISNETLDAALTRLYTSLVQLGYFDPAEGQPLRSLGWDDVATSEAEE
LAKTVAIQGTVLLKNIDWTLPLKANGTLALIGPFINFTTELQSNYAGPAKHIPTMIEAAE
RLGYNVLTAPGTEVNSTSTDGFDDALAIAAEADALIFFGGIDNTVEEESLDRTRIDWPGN
QEELILELAELGRPLTVVQFGGGQVDDSALLASAGVGAIVWAGYPSQAGGAGVFDVLTGK
AAPAGRLPITQYPKSYVDEVPMTDMNLQPGTDNPGRTYRWYEDAVLPFGFGLHYTTFNVS
WAKKAFGPYDAATLARGKNPSSNIVDTFSLAVTNTGDVASDYVALVFASAPELGAQPAPI
KTLVGYSRASLIKPGETRKVDVEVTVAPLTRATEDGRVVLYPGEYTLLVDVNDEYPTAKF
EIKGDVQVLEKFPLSGNDSD

In the present invention, the terms "sequence identity", "identity" and "similarity" are considered equivalent and can be used interchangeably. The term "sequence identity" (or its grammatical equivalents) means that a particular sequence has at least a certain percentage of amino acid residues identical to those in a specified reference sequence using an alignment algorithm. The degree of identity can be determined by, for example, the Clustal method, the Wilbur-Lipman method, the GAG program, including GAP, BLAST or BLASTN, EMBOSS Needle, FASTA, etc. Furthermore, the Smith Waterman algorithm can be used in order to determine the degree of identity between two sequences.

An example of an algorithm that is suitable for determining sequence similarity is the BLAST algorithm, which is described in Altschul, et al, J. Mol. Biol. 215:403-410 (1990). Software for performing BLAST analyses is publicly available through the National Center for Biotechnology Information.

Recombinant beta-xylosidases comprising an amino acid sequence having at least 70% sequence identity to SEQ ID NO: 1 may be obtained from a filamentous fungus, such as, *Acremonium, Aspergillus, Aureobasidium, Cryptococcus, Gibberella, Filibasidium, Fusarium, Humicola, Magnaporthe, Mucor, Neocallimastix, Neurospora, Paecilomyces, Penicillium, Piromyces, Schizophyllum, Talaromyces, Thermoascus, Thielavia, Tolypocladium,* or *Trichoderma.* In a more preferred embodiment, the recombinant beta-xylosidase is an *Aspergillus aculeatus, A. awamori, A. foetidus, A. fumigatus, A. japonicus, A. nidulans, A. niger, A. oryzae, F. bactridioides, F. cerealis, F. crookwellense, F. culmorum, F. graminearum, F. graminum, F. heterosporum, F. negundi, F. oxysporum, F. pseudograminearum, F. reticulatum, F. roseum, F. sambucinum, F. sarcochroum, F. sporotrichioides, F. sulphureum, F. torulosum, F. trichothecioides, F. venenatum, G. zeae, H. insolens, H. lanuginosa, M. miehei, N. crassa, P. purpurogenum, T. harzianum, T. koningii, T. longibrachiatum, T. reesei,* or *T. viride* beta-xylosidase.

In a more preferred embodiment, the recombinant beta-xylosidase enzyme comprises an amino acid sequence having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 1.

In a more preferred embodiment, the recombinant beta-xylosidase enzyme comprises, or consists of, the amino acid sequence SEQ ID NO: 1. The sequence SEQ ID NO: 1 corresponds to the mature beta-xylosidase enzyme from the fungus *A. nidulans* named AnBxl. An example of a beta-xylosidase enzyme comprising the amino acid sequence SEQ ID NO: 1 is the polypeptide of SEQ ID NO: 7 corresponding to the preprotein of the beta-xylosidase enzyme from *A. nidulans,* wherein the amino acids 1 to 23 refer to signal peptide.
SEQ ID NO: 7:

MAVFKSWNLALLSSLFIPALCQSNYPDCTTGPLSELPICDTSLSPLERAKSLVSALTLEE
KINNTGHEAAGSSRLGLPAYNWWNEALHGVAEKHGVSFEESGDFSYATSFPAPIVLGAAF
NDALIRRVAEIISTEARAFSNSDHAGIDYWTPNVNPFKDPRWGRGQETPGEDPLHCSRYV
KEFVGGLQGDDPEKPKVVATCKHLAAYDLEEWGGVSRFEFDAKVSAVDLLEYYLPPFKTC

AVDASVGAFMCSYNALNGVPACADRYLLQTVLREHWGWEGPGHWVTGDCGAVERIQTYHH
YVESGPEAAAAALNAGVDLDCGTWLPSYLGEAERQGLISNETLDAALTRLYTSLVQLGYF
DPAEGQPLRSLGWDDVATSEAEELAKTVAIQGTVLLKNIDWTLPLKANGTLALIGPFINF
TTELQSNYAGPAKHIPTMIEAAERLGYNVLTAPGTEVNSTSTDGFDDALAIAAEADALIF
FGGIDNTVEEESLDRTRIDWPGNQEELILELAELGRPLTVVQFGGGQVDDSALLASAGVG
AIVWAGYPSQAGGAGVFDVLTGKAAPAGRLPITQYPKSYVDEVPMTDMNLQPGTDNPGRT
YRWYEDAVLPFGFGLHYTTFNVSWAKKAFGPYDAATLARGKNPSSNIVDTFSLAVTNTGD
VASDYVALVFASAPELGAQPAPIKTLVGYSRASLIKPGETRKVDVEVTVAPLTRATEDGR
VVLYPGEYTLLVDVNDEYPTAKFEIKGDVQVLEKFPLSGNDSD

The recombinant beta-xylosidase enzyme can be bound/linked to a signal peptide which directs the enzyme into the cell's secretory pathway. Thus, in another preferred embodiment, the beta-xylosidase enzyme comprises a signal peptide or signal sequence. Said signal peptide may be heterologous to the enzyme, i.e. the signal peptide and the enzyme comes from different microorganisms, or be the native signal sequence of the beta-xylosidase enzyme, i.e. the signal peptide of the beta-xylosidase enzyme from *A. nidulans.*

When the recombinant beta-xylosidase enzyme comprises, or consist of, the amino acid sequence SEQ ID NO: 7, the host cell is not an *Aspergillus nidulans* cell.

In a preferred embodiment, the signal peptide is heterologous to the beta-xylosidase enzyme.

Effective signal peptide sequences for bacterial host cells are the signal peptide obtained from *Bacillus stearothermophilus* maltogenic amylase, *Bacillus licheniformis* subtilisin, *B. licheniformis beta-lactamase, B. stearothermophilus alpha-amylase, B. stearothermophilus* neutral proteases (*nprT, nprS, nprM*), *Bacillus subtilis prsA,* etc. Effective signal peptide sequences for filamentous fungal host cells are the signal peptide obtained from *Aspergillus niger* neutral amylase, *A. niger* glucoamylase A (SEQ ID NO: 12), *Aspergillus shirousami* glucoamylase A, *Aspergillus kawachii* glucoamylase I, *Aspergillus oryzae* TAKA amylase, *Humicola insolens* cellulase, *H. insolens* endoglucanase V, *Humicola lanuginosa* lipase, and *Rhizomucor miehei* aspartic proteinase.

### SEQ ID NO: 12: MSFRSLLALSGLVCTGLA

In a preferred embodiment, the signal peptide is the glucoamylase A signal peptide, preferably, the glucoamylase A signal peptide from *Aspergillus awamori,* more preferably, the signal peptide comprises the sequence SEQ ID NO: 2.
SEQ ID NO: 2: MSFRSLLALSGLVCSGLA

The resulting polypeptide of binding the signal peptide to an amino acid sequence having at least 70% sequence identity to SEQ ID NO: 1 is a fusion polypeptide or fusion protein, wherein the signal peptide sequence is foreign to, or heterologous to, said an amino acid sequence having at least 70% sequence identity to SEQ ID NO: 1. The meaning of the terms "foreign" and "heterologous" has been explained above.

Thus, the beta-xylosidase enzyme may be a fusion polypeptide or cleavable fusion polypeptide in which a signal peptide sequence is fused at the N-terminus of the amino acid sequence having at least 70% sequence identity to SEQ ID NO: 1. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

In a particular embodiment, the beta-xylosidase enzyme is a fusion protein having the amino acid sequence SEQ ID NO: 3, comprising the glucoamylase A signal peptide from *A. awamori* of sequence SEQ ID NO: 2 bound to the an amino acid sequence of SEQ ID NO: 1.
SEQ ID NO: 3:

MSFRSLLALSGLVCSGLANYPDCTTGPLSELPICDTSLSPLERAKSLVSALTLEEKINNT
GHEAAGSSRLGLPAYNWWNEALHGVAEKHGVSFEESGDFSYATSFPAPIVLGAAFNDALI
RRVAEIISTEARAFSNSDHAGIDYWTPNVNPFKDPRWGRGQETPGEDPLHCSRYVKEFVG
GLQGDDPEKPKVVATCKHLAAYDLEEWGGVSRFEFDAKVSAVDLLEYYLPPFKTCAVDAS
VGAFMCSYNALNGVPACADRYLLQTVLREHWGWEGPGHWVTGDCGAVERIQTYHHYVESG
PEAAAAALNAGVDLDCGTWLPSYLGEAERQGLISNETLDAALTRLYTSLVQLGYFDPAEG
QPLRSLGWDDVATSEAEELAKTVAIQGTVLLKNIDWTLPLKANGTLALIGPFINFTTELQ
SNYAGPAKHIPTMIEAAERLGYNVLTAPGTEVNSTSTDGFDDALAIAAEADALIFFGGID
NTVEEESLDRTRIDWPGNQEELILELAELGRPLTVVQFGGGQVDDSALLASAGVGAIVWA
GYPSQAGGAGVFDVLTGKAAPAGRLPITQYPKSYVDEVPMTDMNLQPGTDNPGRTYRWYE
DAVLPFGFGLHYTTFNVSWAKKAFGPYDAATLARGKNPSSNIVDTFSLAVTNTGDVASDY
VALVFASAPELGAQPAPIKTLVGYSRASLIKPGETRKVDVEVTVAPLTRATEDGRVVLYP
GEYTLLVDVNDEYPTAKFEIKGDVQVLEKFPLSGNDSD

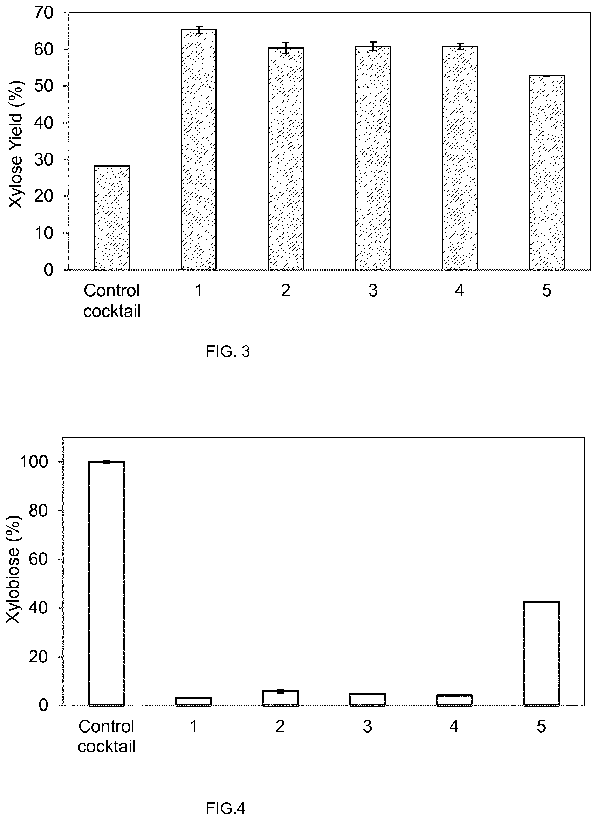

As it will be shown in examples below, the highest yield of released xylose and hydrolyzed xylobiose during the hydrolytic process of biomass was obtained when the host cell of the invention expressed this SEQ ID NO: 3 (see Figure 3 & 4). In a particular embodiment, the beta-xylosidase enzyme consists of the sequence SEQ ID NO: 3.

As explained above, the host cell of the invention expresses a nucleotide sequence encoding the recombinant beta-xylosidase enzyme disclosed herein. Nucleotide sequences encoding these recombinant beta-xylosidases can encode the mature polypeptide (such as, SEQ ID NO: 1) or a preprotein thereof (such as SEQ ID NO: 3) comprising a signal peptide (such as SEQ ID NO: 2) linked to the amino acid sequence of said mature enzyme (such as SEQ ID NO: 1). As the skilled person in the art understand, the preprotein of sequence SEQ ID NO: 3 will have to be subsequently processed by the cell giving rise to the mature polypeptide SEQ ID NO: 1.

Thus in another preferred embodiment, the nucleotide sequence encoding the beta-xylosidase enzyme disclosed herein comprises the sequence SEQ ID NO: 4 which preferably is bound to a nucleotide sequence encoding a signal peptide. In a more preferred embodiment, the nucleotide sequence encoding the signal peptide comprises the sequence SEQ ID NO: 5. In a most preferred embodiment, the nucleotide sequence encoding the beta-xylosidase enzyme comprises the sequence SEQ ID NO: 6.
SEQ ID NO: 4: 
AACTACCCCGACTGCACCACCGGCCCCCTCTCCGAGCTGCCCATCTGCGACACCTCCCTCTC
CCCCCTGGAGCGCGCCAAGTCCCTCGTCTCCGCCCTCACCCTGGAGGAGAAGATCAACAACA
CCGGCCACGAGGCCGCCGGCTCCTCCCGCCTCGGCCTCCCCGCCTACAACTGGTGGAACGA
GGCCCTCCACGGCGTCGCCGAGAAGCACGGCGTCTCCTTCGAGGAGTCCGGCGACTTCTCCT
ACGCCACCTCCTTCCCCGCCCCCATCGTCCTCGGCGCCGCCTTCAACGACGCCCTCATCCGC
CGCGTCGCCGAGATCATCTCCACCGAGGCCCGCGCCTTCTCCAACTCCGACCACGCCGGCAT
CGACTACTGGACCCCCAACGTCAACCCCTTCAAGGACCCCCGCTGGGGCCGCGGCCAGGAGA
CCCCCGGCGAGGACCCCCTCCACTGCTCCCGCTACGTCAAGGAGTTCGTCGGCGGCCTCCAG
GGCGACGACCCCGAGAAGCCCAAGGTCGTCGCCACCTGCAAGCACCTCGCCGCCTACGACCT
GGAGGAGTGGGGCGGCGTCTCCCGCTTCGAGTTCGACGCCAAGGTCTCCGCCGTGGACCTCC
TGGAGTACTACCTCCCCCCCTTCAAGACCTGCGCCGTGGACGCCTCCGTCGGCGCCTTCATG
TGCTCCTACAACGCCCTCAACGGCGTCCCCGCCTGCGCCGACCGCTACCTCCTCCAGACCGT
CCTCCGCGAGCACTGGGGCTGGGAGGGCCCCGGCCACTGGGTCACCGGCGACTGCGGCGCCG
TCGAGCGCATCCAGACCTACCACCACTACGTCGAGTCCGGCCCCGAGGCCGCCGCCGCCGCC
CTCAACGCCGGCGTGGACCTCGACTGCGGCACCTGGCTCCCCTCCTACCTCGGCGAGGCCGA
GCGCCAGGGCCTCATCTCCAACGAGACCCTCGACGCCGCCCTCACCCGCCTCTACACCTCCC
TCGTCCAGCTCGGCTACTTCGACCCCGCCGAGGGCCAGCCCCTCCGCTCCCTCGGCTGGGAC
GACGTCGCCACCTCCGAGGCCGAGGAGCTGGCCAAGACCGTCGCCATCCAGGGCACCGTCCT
CCTCAAGAACATCGACTGGACCCTCCCCCTCAAGGCCAACGGCACCCTCGCCCTCATCGGCC
CCTTCATCAACTTCACCACCGAGCTGCAGTCCAACTACGCCGGCCCCGCCAAGCACATCCCC
ACCATGATCGAGGCCGCCGAGCGCCTCGGCTACAACGTCCTCACCGCCCCCGGCACCGAGGT
CAACTCCACCTCCACCGACGGCTTCGACGACGCCCTCGCCATCGCCGCCGAGGCCGACGCCC
TCATCTTCTTCGGCGGCATCGACAACACCGTCGAGGAGGAGTCCCTCGACCGCACCCGCATC
GACTGGCCCGGCAACCAGGAGGAGCTGATCCTGGAGCTGGCCGAGCTGGGCCGCCCCCTCAC
CGTCGTCCAGTTCGGCGGCGGCCAGGTGGACGACTCCGCCCTCCTCGCCTCCGCCGGCGTCG
GCGCCATCGTCTGGGCCGGCTACCCCTCCCAGGCCGGCGGCGCCGGCGTCTTCGACGTCCTC
ACCGGCAAGGCCGCCCCCGCCGGCCGCCTCCCCATCACCCAGTACCCCAAGTCCTACGTGGA
CGAGGTCCCCATGACCGACATGAACCTCCAGCCCGGCACCGACAACCCCGGCCGCACCTACC
GCTGGTACGAGGACGCCGTCCTCCCCTTCGGCTTCGGCCTCCACTACACCACCTTCAACGTC
TCCTGGGCCAAGAAGGCGTTCGGCCCCTACGACGCCGCCACCCTCGCCCGCGGCAAGAACCC
CTCCTCCAACATCGTGGACACCTTCTCCCTCGCCGTCACCAACACCGGCGACGTCGCCTCCG
ACTACGTCGCCCTCGTCTTCGCCTCCGCCCCCGAGCTGGGCGCCCAGCCCGCCCCCATCAAG
ACCCTCGTCGGCTACTCCCGCGCCTCCCTCATCAAGCCCGGCGAGACCCGCAAGGTGGACGT
CGAGGTCACCGTCGCCCCCCTCACCCGCGCCACCGAGGACGGCCGCGTCGTCCTCTACCCCG
GCGAGTACACCCTCCTCGTGGACGTCAACGACGAGTACCCCACCGCCAAGTTCGAGATCAAG
GGCGACGTCCAGGTCCTGGAGAAGTTCCCCCTCTCCGGCAACGACTCCGACTAA
SEQ ID NO: 5:
   ATGTCGTTCCGATCTCTTCTCGCCCTGAGCGGCCTTGTCTGCTCGGGGTTGGCA
SEQ ID NO: 6:

ATGTCGTTCCGATCTCTTCTCGCCCTGAGCGGCCTTGTCTGCTCGGGGTTGGCAAACTACCC
CGACTGCACCACCGGCCCCCTCTCCGAGCTGCCCATCTGCGACACCTCCCTCTCCCCCCTGG
AGCGCGCCAAGTCCCTCGTCTCCGCCCTCACCCTGGAGGAGAAGATCAACAACACCGGCCAC
GAGGCCGCCGGCTCCTCCCGCCTCGGCCTCCCCGCCTACAACTGGTGGAACGAGGCCCTCCA
CGGCGTCGCCGAGAAGCACGGCGTCTCCTTCGAGGAGTCCGGCGACTTCTCCTACGCCACCT
CCTTCCCCGCCCCCATCGTCCTCGGCGCCGCCTTCAACGACGCCCTCATCCGCCGCGTCGCC
GAGATCATCTCCACCGAGGCCCGCGCCTTCTCCAACTCCGACCACGCCGGCATCGACTACTG
GACCCCCAACGTCAACCCCTTCAAGGACCCCCGCTGGGGCCGCGGCCAGGAGACCCCCGGCG
AGGACCCCCTCCACTGCTCCCGCTACGTCAAGGAGTTCGTCGGCGGCCTCCAGGGCGACGAC
CCCGAGAAGCCCAAGGTCGTCGCCACCTGCAAGCACCTCGCCGCCTACGACCTGGAGGAGTG
GGGCGGCGTCTCCCGCTTCGAGTTCGACGCCAAGGTCTCCGCCGTGGACCTCCTGGAGTACT
ACCTCCCCCCCTTCAAGACCTGCGCCGTGGACGCCTCCGTCGGCGCCTTCATGTGCTCCTAC
AACGCCCTCAACGGCGTCCCCGCCTGCGCCGACCGCTACCTCCTCCAGACCGTCCTCCGCGA
GCACTGGGGCTGGGAGGGCCCCGGCCACTGGGTCACCGGCGACTGCGGCGCCGTCGAGCGCA
TCCAGACCTACCACCACTACGTCGAGTCCGGCCCCGAGGCCGCCGCCGCCGCCCTCAACGCC
GGCGTGGACCTCGACTGCGGCACCTGGCTCCCCTCCTACCTCGGCGAGGCCGAGCGCCAGGG
CCTCATCTCCAACGAGACCCTCGACGCCGCCCTCACCCGCCTCTACACCTCCCTCGTCCAGC
TCGGCTACTTCGACCCCGCCGAGGGCCAGCCCCTCCGCTCCCTCGGCTGGGACGACGTCGCC
ACCTCCGAGGCCGAGGAGCTGGCCAAGACCGTCGCCATCCAGGGCACCGTCCTCCTCAAGAA
CATCGACTGGACCCTCCCCCTCAAGGCCAACGGCACCCTCGCCCTCATCGGCCCCTTCATCA
ACTTCACCACCGAGCTGCAGTCCAACTACGCCGGCCCCGCCAAGCACATCCCCACCATGATC
GAGGCCGCCGAGCGCCTCGGCTACAACGTCCTCACCGCCCCCGGCACCGAGGTCAACTCCAC
CTCCACCGACGGCTTCGACGACGCCCTCGCCATCGCCGCCGAGGCCGACGCCCTCATCTTCT
TCGGCGGCATCGACAACACCGTCGAGGAGGAGTCCCTCGACCGCACCCGCATCGACTGGCCC
GGCAACCAGGAGGAGCTGATCCTGGAGCTGGCCGAGCTGGGCCGCCCCCTCACCGTCGTCCA
GTTCGGCGGCGGCCAGGTGGACGACTCCGCCCTCCTCGCCTCCGCCGGCGTCGGCGCCATCG
TCTGGGCCGGCTACCCCTCCCAGGCCGGCGGCGCCGGCGTCTTCGACGTCCTCACCGGCAAG
GCCGCCCCCGCCGGCCGCCTCCCCATCACCCAGTACCCCAAGTCCTACGTGGACGAGGTCCC
CATGACCGACATGAACCTCCAGCCCGGCACCGACAACCCCGGCCGCACCTACCGCTGGTACG
AGGACGCCGTCCTCCCCTTCGGCTTCGGCCTCCACTACACCACCTTCAACGTCTCCTGGGCC
AAGAAGGCGTTCGGCCCCTACGACGCCGCCACCCTCGCCCGCGGCAAGAACCCCTCCTCCAA
CATCGTGGACACCTTCTCCCTCGCCGTCACCAACACCGGCGACGTCGCCTCCGACTACGTCG
CCCTCGTCTTCGCCTCCGCCCCCGAGCTGGGCGCCCAGCCCGCCCCCATCAAGACCCTCGTC
GGCTACTCCCGCGCCTCCCTCATCAAGCCCGGCGAGACCCGCAAGGTGGACGTCGAGGTCAC
CGTCGCCCCCCTCACCCGCGCCACCGAGGACGGCCGCGTCGTCCTCTACCCCGGCGAGTACA
CCCTCCTCGTGGACGTCAACGACGAGTACCCCACCGCCAAGTTCGAGATCAAGGGCGACGTC
CAGGTCCTGGAGAAGTTCCCCCTCTCCGGCAACGACTCCGACTAA

Nucleic acid sequences encoding beta-xylosidases can be included in a genetic construct, preferably in an expression vector. Said genetic construct may further comprise one or more regulatory sequences of gene expression, such as promoters, terminators, etc.

In accordance with the present invention, "nucleic acid sequence" or "polynucleotide" or "nucleotide sequence" is a nucleic acid molecule (polynucleotide) that has been removed from its natural milieu (i.e., that has been subject to human manipulation) and can include DNA, RNA, or derivatives of either DNA or RNA, including cDNA. The nucleotide sequence of the present invention can be or not chemically or biochemically modified and can be artificially performed by means of cloning and selection methods or by sequencing.

The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for the expression of a coding sequence of the recombinant beta-xylosidase.

The term "control sequences" is defined herein to include all components which are necessary or advantageous for the expression of a polynucleotide encoding a recombinant beta-xylosidase of the present invention. Each control sequence may be native or heterologous to the nucleotide sequence encoding the recombinant beta-xylosidase. Such control sequences include, but are not limited to, a leader sequence, polyadenylation sequence, propeptide sequence, promoter, signal peptide sequence, and transcription terminator.

The control sequence may be a promoter,i.e. a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *B. licheniformis* alpha-amylase gene (*amyL*), *B. licheniformis* penicillinase gene (*penP*), *B. stearothermophilus* maltogenic amylase gene (amyM), *B. subtilis* levansucrase gene (sacB), *B. subtilis xylA* and *xylB* genes, *Bacillus thuringiensis cryIIIA* gene (Agaisse and Lereclus, 1994, Molecular Microbiology 13: 97-107), *Escherichia coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al*., 1989, supra. Examples of tandem promoters are disclosed in WO 99/43835.

Examples of suitable promoters for directing transcription of the nucleic acid constructs of the present invention in a filamentous fungal host cell are promoters obtained from the genes for *A. nidulans* acetamidase, *A. niger* neutral alpha-amylase, *A. niger* acid stable alpha-amylase, *A. niger* or A. awamori glucoamylase (*glaA*), *A. oryzae* TAKA amylase, *A. oryzae* alkaline protease, *A. oryzae* triose phosphate isomerase, *F. oxysporum* trypsin-like protease (WO 96/00787), *F. venenatum* amyloglucosidase (WO 00/56900), *F. venenatum* Dania (WO 00/56900), *F. venenatum* Quinn (WO 00/56900), Rhizomucor *miehei* lipase, *R. miehei* aspartic proteinase, *T. reesei* beta-glucosidase, *T. reesei* cellobiohydrolase I, *T. reesei* cellobiohydrolase II, *T. reesei* endoglucanase I, *T. reesei* endoglucanase II, *T. reesei* endoglucanase III, *T. reesei* endoglucanase V, *T. reesei* xylanase I, *T. reesei* xylanase II, *T. reesei* xylanase III, *T. reesei* beta-xylosidase, and *T. reesei* translation elongation factor, as well as the NA2-tpi promoter (a modified promoter from an *Aspergillus* neutral alpha-amylase gene in which the untranslated leader sequence has been replaced by an untranslated leader sequence from an *Aspergillus* triose phosphate isomerase gene; non-limiting examples include modified promoters from an *A. niger* neutral alpha-amylase gene in which the untranslated leader has been replaced by an untranslated leader from an *A. nidulans* or *A. oryzae* triose phosphate isomerase gene); and mutant, truncated, and hybrid promoters thereof. Other promoters are described in U.S. Pat. No. 6,011,147. In a preferred embodiment, the promoter is the promoter from *A. nidulans* beta-xylosidase.

In a yeast host cell, useful promoters are obtained from the genes for *Saccharomyces cerevisiae* enolase (*ENO-1*), *S. cerevisiae* galactokinase (*GAL1*), *S. cerevisiae* alcohol dehydrogenase/glyceraldehyde-3-phosphate dehydrogenase (*ADH1, ADH2*/*GAP*), *S. cerevisiae* triose phosphate isomerase (TPI), *S. cerevisiae* metallothionein (CUP1), and *S. cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *B. licheniformis* alpha-amylase (*amyL*), and *E. coli* ribosomal RNA (*rrnB*).

Preferred terminators for filamentous fungal host cells are obtained from the genes for *A. nidulans* acetamidase, *A. nidulans* anthranilate synthase, *A. niger* glucoamylase, *A. niger* alpha-glucosidase, *A. oryzae* TAKA amylase, *F. oxysporum* trypsin-like protease, *T. reesei* beta-glucosidase, *T. reesei* cellobiohydrolase I, *T. reesei* cellobiohydrolase II, *T. reesei* endoglucanase I, *T. reesei* endoglucanase II, *T. reesei* endoglucanase III, *T. reesei* endoglucanase V, *T. reesei* xylanase I, *T. reesei* xylanase II, *T. reesei* xylanase III, *T. reesei* beta-xylosidase, and *T. reesei* translation elongation factor.

Preferred terminators for yeast host cells are obtained from the genes for *S. cerevisiae* enolase, *S. cerevisiae* cytochrome C(*CYC1*), and *S. cerevisiae* glyceraldehyde-3-phosphate dehydrogenase. Other useful terminators for yeast host cells are described by Romanos *et al.,* 1992, cited *ad supra.*

The control sequence may also be an mRNA stabilizer region downstream of a promoter and upstream of the coding sequence of a gene which increases expression of the gene.

Examples of suitable mRNA stabilizer regions are obtained from a *B. thuringiensis cryIIIA* gene (WO 94/25612) and a *B. subtilis* SP82 gene (Hue et al., 1995, Journal of Bacteriology 177: 3465-3471).

The control sequence may also be a leader sequence, a nontranslated region of an mRNA that is important for translation by the host cell. The leader sequence is operably linked to the 5'-terminus of the polynucleotide encoding the polypeptide. Any leader that is functional in the host cell may be used.

Preferred leader sequences for filamentous fungal host cells are obtained from the genes for *A. oryzae* TAKA amylase and *A. nidulans* triose phosphate isomerase.

Suitable leader sequences for yeast host cells are obtained from the genes for S. cerevisiae enolase (*ENO-1*), *S. cerevisiae* 3-phosphoglycerate kinase, *S. cerevisiae* alpha-factor, and *S. cerevisiae* alcohol dehydrogenase/glyceraldehyde-3-phosphate dehydrogenase (*ADH2*/*GAP*).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *A. nidulans* anthranilate synthase, *A. niger* glucoamylase, *A. niger* alpha-glucosidase *A. oryzae* TAKA amylase, and *F. oxysporum* trypsin-like protease.
Useful polyadenylation sequences for yeast host cells are described by Guo and Sherman, 1995, Mol. Cellular Biol. 15: 5983-5990.

At a minimum, the control sequences comprise a signal peptide sequence as explained previously, and preferably a promoter, and transcriptional and translational stop signals. The control sequences may be provided with linkers for the purpose of introducing specific restriction sites facilitating ligation of the control sequences with the coding region of the nucleotide sequence encoding the recombinant beta-xylosidase. The term "operably linked" denotes herein a configuration in which a control sequence is placed at an appropriate position relative to the coding sequence of the polynucleotide sequence such that the control sequence directs the expression of the coding sequence of the recombinant beta-xylosidase.

The nucleotide sequence encoding the beta-xylosidase described herein may be expressed by inserting the nucleotide sequence or a nucleic acid construct comprising the sequence into an appropriate vector for expression.

The term "expression vector" is defined herein as a linear or circular DNA molecule that comprises a polynucleotide encoding the recombinant beta-xylosidase as disclosed herein, and which is operably linked to additional nucleotides that provide for its expression. Said vector comprising a polynucleotide encoding the recombinant beta-xylosidase is introduced into a host cell so that the vector is maintained as a chromosomal integrant or as a self-replicating extra-chromosomal vector.

In creating the expression vector, the coding sequence is located in the vector so that the coding sequence is operably linked with the appropriate control sequences for expression. The expression vectors referred to in the present invention comprise a polynucleotide encoding the beta-xylosidase described herein, a promoter, and transcriptional and translational stop signals. The various nucleic acids and control sequences described herein may be joined together to produce a recombinant expression vector which may include one or more convenient restriction sites to allow for insertion or substitution of the nucleotide sequence encoding the enzyme at such sites.

The recombinant expression vector may be any vector (e.g., a plasmid or virus) which can be conveniently subjected to recombinant DNA procedures and can bring about expression of the nucleotide sequence. The choice of the vector will typically depend on the compatibility of the vector with the host cell into which the vector is to be introduced.

The vectors may be linear or closed circular plasmids. The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors used in the present invention preferably contain one or more selectable markers which permit easy selection of transformed, transfected, transduced, or the like cells. A selectable marker is a gene the product of which provides for biocide or viral resistance, resistance to heavy metals, prototrophy to auxotrophs, and the like. Selectable markers for use in a filamentous fungal host cell include, but are not limited to, *amdS* (acetamidase), *argB* (ornithine carbamoyltransferase), bar (phosphinothricin acetyltransferase), *hph* (hygromycin phosphotransferase), *niaD* (nitrate reductase), *pyrG* (orotidine-5'-phosphate decarboxylase), *pyr5, cysC* (sulfate adenyltransferase), and *trpC* (anthranilate synthase), as well as equivalents thereof.

The vectors used in the present invention preferably contain an element(s) that permits integration of the vector into the host cell's genome or autonomous replication of the vector in the cell independent of the genome. For integration into the host cell genome, the vector may rely on the polynucleotide's sequence encoding the beta-xylosidase enzyme or any other element of the vector for integration into the genome by homologous or non-homologous recombination. Alternatively, the vector may contain additional nucleotide sequences for directing integration by homologous recombination into the genome of the host cell at a precise location(s) in the chromosome(s).

For autonomous replication, the vector may further comprise an origin of replication enabling the vector to replicate autonomously in the host cell in question. The origin of replication may be any plasmid replicator mediating autonomous replication which functions in a cell. The term "origin of replication" or "plasmid replicator" is defined herein as a nucleotide sequence that enables a plasmid or vector to replicate *in vivo.* Examples of origins of replication useful in a filamentous fungal cell are *AMAI* and *ANSI.*

More than one copy of a nucleotide sequence encoding the beta-xylosidase of the present invention may be inserted into the host cell to increase the production of the gene product. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected by cultivating the cells in the presence of the appropriate selectable agent. The procedures used to ligate the elements described above to construct the recombinant expression vectors referred to in the present invention are well known to one skilled in the art.

The term "expression" includes any step involved in the production of the recombinant beta-xylosidase including, but not limited to, transcription, post-transcriptional modification, translation, post-translational modification, and secretion.

As explained above, the host cell of the invention expresses a nucleotide sequence encoding a functional recombinant beta-xylosidase enzyme and it is capable of secreting it to the extracellular medium. The term "functional" means that the expressed enzyme retains its capacity to hydrolyse xylan oligomers to xylose. This activity can be measured by means of any suitable method known in the state of the art to assess the beta-xylosidase activity, preferably by means of the method described below in examples of the present invention. The most usual detection method involves the use of the chromogenic substrate para-nitrophenyl beta-D-xylopyranoside (hereinafter pNXP). A preferred method involves the measurement of excision of xylobiose to xylose by HPLC.

The expression of the beta-xylosidase in the host cell of the invention may be performed by means of any method known in the art, such as transformation of a suitable host cell with a nucleotide sequence encoding the recombinant beta-xylosidase, or a genetic construction comprising said nucleotide sequence, and cultivation of the transformed host cell under conditions which induce the expression of said nucleotide sequence in order to obtained the secreted enzyme.

The host cell can be cultivated in a nutrient medium suitable for production of the recombinant beta-xylosidase using methods well known in the art. For example, the cell may be cultivated by shake flask cultivation, and small-scale or large-scale fermentation (including continuous, batch, fed-batch, or solid state fermentations) in laboratory or industrial bioreactor performed in a suitable medium and under conditions allowing the beta-xylosidase to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the beta-xylosidase is secreted into the nutrient medium, the beta-xylosidase can be recovered directly from the medium.

The recombinant beta-xylosidase expressed may be detected using methods known in the art that are specific for the polypeptides. These detection methods may include use of specific antibodies, formation of an enzyme product, or disappearance of an enzyme substrate.

The resulting beta-xylosidase may be recovered using methods known in the art. For example, the beta-xylosidase may be recovered from the nutrient medium by conventional procedures including, but not limited to, centrifugation, filtration, extraction, spray-drying, evaporation, or precipitation.

The beta-xylosidases produced in the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction, in order to obtain substantially pure beta-xylosidase that can be included in an enzymatic composition together with other cellulolytic enzymes.

Thus, a second aspect of the invention refers to a recombinant beta-xylosidase enzyme expressed by the host cell of the invention. Preferably, said recombinant beta-xylosidase enzyme comprise, or consists of, the amino acid sequence SEQ ID NO: 7 (preprotein beta-xylosidase from *A. nidulans*), SEQ ID NO: 1 (mature beta-xylosidase from *A. nidulans*) or SEQ ID NO: 3 (SPCG+mature beta-xylosidase from *A. nidulans*), more preferably SEQ ID NO: 3.

A third aspect of the invention refers to a composition comprising the recombinant beta-xylosidase enzyme produced by means of the host cell of the invention, preferably the enzyme comprising, or consisting of, the sequence SEQ ID NO: 3 (hereinafter "composition of the invention"), and/or the host cell of the invention. This composition of the invention may further comprise one or more of other enzymatic activities, such as aminopeptidase, amylase, carbohydrase, carboxypeptidase, catalase, cellulase, such as arabinofuranosidase, xyloglucanase, polysaccharide mono-oxygenases (previously known as glycosyl-hydrolases family 61 or GH61), endoglucanases, beta-glucosidases and/or cellobiohydrolases; chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase, or any combination thereof. The additional enzyme(s) may be produced, for example, by a microorganism belonging to the genus *Aspergillus,* such as *A. aculeatus, A. awamori, A. fumigatus, A. foetidus, A. japonicus, A. nidulans, A. niger,* or *A. oryzae; Fusarium,* such as *F.* bactridioides, *F. cerealis, F. crookwellense, F. culmorum, F. graminearum, F. graminum, F. heterosporum, F. negundi, F. oxysporum, F. pseudograminearum, F. reticulatum, F. roseum, F. sambucinum, F. sarcochroum, F. sulphureum, F. toruloseum, F. trichothecioides,* or *F. venenatum; Gibberella,* such as *G. zeae; Humicola,* such as *H. insolens* or *H. lanuginosa; Trichoderma,* such as *T. harzianum, T. koningii, T. longibrachiatum, T. reesei,* or *T. viride;* or *Myceliophthora,* such as *M. heterotalica* or *M. thermophila.*

In a preferred embodiment, the composition of the invention further comprises one or more of other cellulolytic enzymes. The term "cellulolytic enzymes" also known as "cellulases", refers to a category of enzymes capable of hydrolysing cellulose (beta-1,4-glucan or beta-D-glucosidic linkages) or hemicellulose to shorter oligosaccharides, cellobiose and/or glucose. Examples of cellulolytic enzymes are, but not limited to, arabinofuranosidase, xyloglucanase, polysaccharide mono-oxygenases, endoglucanases, beta-glucosidases, cellobiohydrolases or endoxylanases. Thus, in a more preferred embodiment, these cellulolytic enzymes are selected from the list consisting of: arabinofuranosidase, xyloglucanase, polysaccharide mono-oxygenases, endoglucanases, beta-glucosidases, cellobiohydrolases, endoxylanases and any combination thereof. These cellulolytic enzymes can derive from the host cell of the invention or other cellulolytic enzymes producers-microorganisms different from the host cell of the invention. Likewise, they can be naturally or recombinantly produced.

Preferably, the composition of the invention comprises the recombinant beta-xylosidase enzyme produced by means of the host cell of the invention, preferably the enzyme comprising, or consisting of, the SEQ ID NO: 1, and other cellulolytic enzymes derived from the host cell of the invention. In a more preferred embodiment, the composition of the invention is an enzymatic mixture obtained by the host cell of the invention. In an even more preferred embodiment, the composition of the invention is an enzymatic mixture obtained by the *M. thermophila* host cell of the invention, preferably *M. thermophila C1,* wherein said cell comprises a nucleotide sequence encoding the recombinant beta-xylosidase enzyme which comprises, or consists of, the amino acid sequence SEQ ID NO: 3.

The term "endoglucanase" or "EG" refers to a group of cellulase enzymes classified as E.C. 3.2.1.4. These enzymes hydrolyse internal beta-1,4 glucosidic bonds of cellulose. Endoglucanase activity may be determined by measuring reduction in substrate viscosity or increase in reducing ends determined by a reducing sugar assay (Zhang et al., 2006, Biotechnology Advances 24: 452-481).

The term "cellobiohydrolase" refers to a protein that catalyzes the hydrolysis of cellulose to cellobiose via an exoglucanase activity, sequentially releasing molecules of cellobiose from the reducing or non-reducing ends of cellulose or cellooligosaccharides. Cellobiohydrolase activity may be determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al. , 1988, Eur. J. Biochem. 170: 575-581.

The term "beta-glucosidase" as used herein refers to an enzyme which catalyses the hydrolysis of a sugar dimer, including but not limited to cellobiose, with the release of a corresponding sugar monomer, used, but not limited, for the synthesis of ethanol. Beta-glucosidase enzyme acts upon beta1->4 bonds linking two glucose or glucose-substituted molecules (i.e., the disaccharide cellobiose). It is an exocellulase with specificity for a variety of beta-D-glycoside substrates. It catalyzes the hydrolysis of terminal non-reducing residues in beta-D-glucosides with release of glucose. Beta-glucosidase activity may be determined using p-nitrophenyl-beta-D-glucopyranoside as substrate according to the procedure of Venturi et al., 2002, J. Basic Microbiol. 42: 55-66.

The term "endoxylanase" refers to an enzyme which catalyzes the endohydrolysis of 1,4-beta-D-xylosidic linkages in xylanes.

The term "arabinofuranosidase" or "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L- arabinofuranosidase is also known as arabinosidase, alpha-arabinosidase, alpha-L- arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present invention, alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 µl for 30 minutes at 40°C followed by arabinose analysis by AMINEX® HPX-87H column chromatography (Bio-Rad Laboratories, Inc., Hercules, CA, USA).

The term "xyloglucanase" refers to any enzyme which has an activity towards the substrate xyloglucan. Examples of useful xyloglucanases are family 12 xyloglucan hydrolyzing endoglucanases, in particular family 12 xyloglucan hydrolyzing endoglucanases, obtained from e.g. *A. aculeatus* as described in WO 94/14953. Another useful example is a xyloglucanase produced by *Trichoderma,* especially EGIII. The xyloglucanase may also be an endoglucanase with xyloglucanase activity and low activity towards insoluble cellulose and high activity towards soluble cellulose, e.g., family 7 endoglucanases obtained from, e.g., H. insolens. The xyloglucanase activity may be measured by means of measuring the residual sugar: A purified source of xyloglucan (e.g. from Megazyme International Ireland, Ltd., Bray, Co. Wicklow, Ireland) is dissolved in a suitable buffer (250 µg xyloglucan in 100 µL buffer) and incubated with 30-400 ng of enzyme for 1 or 20 hours. The increase in reducing sugar is determined according to the procedure of Somogyi using glucose for calibration.

The term "polysaccharide mono-oxygenases", formerly classified as GH61 and CBM33 enzymes in the CAZy database, refers to enzymes having cellulolytic enhancing activity and means a polypeptide that catalyzes the enhancement of the hydrolysis of a cellulosic material. For purposes of the present invention, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in pretreated corn stover (PCS), wherein total protein is comprised of 50-99.5% w/w cellulolytic enzyme protein and 0.5-50% w/w protein of a GH61 polypeptide having cellulolytic enhancing activity for 1-7 days at a suitable temperature, such as 40°C- 80°C, e.g., 50°C, 55°C, 60°C, 65°C, or 70°C, and a suitable pH, such as 4-9, e.g., 4.5, 5.0, 5.5, 6.0, 6.5, 7.0, 7.5, 8.0, or 8.5, compared to a control hydrolysis with equal total protein loading without cellulolytic enhancing activity (1-50 mg of cellulolytic protein/g of cellulose in PCS).

The composition of the invention may be prepared in accordance with methods known in the art and may be in the form of a liquid or a dry composition. For instance, the composition may be in the form of a granulate or a microgranulate. The enzymes to be included in the composition may be stabilized in accordance with methods known in the art.

As stated above, the host cell of the invention expresses a recombinant beta-xylosidase enzyme comprising an amino acid sequence having at least 70% sequence identity to SEQ ID NO: 1, preferably the beta-xylosidase enzyme from A. *nidulans,* more preferably, the beta-xylosidase enzyme from *A. nidulans* of SEQ ID NO: 1, which is capable of degrading xylan oligomers to xylose when secreted to the extracellular medium. This host cell is capable of secrete this enzyme to the medium together with other native or recombinantly produced cellulolytic enzymes, being thus useful for the optimization of the hydrolysis step of biomass into fermentable sugars.

Therefore, a fourth aspect the invention relates to the use of the host cell of the invention, the use of the recombinant beta-xylosidase enzyme produced by means of the host cell of the invention or the use of the composition of the invention for the degradation of biomass.

The term "biomass" means the biodegradable fraction of products, waste and residues from biological origin from agriculture (including vegetal, such as crop residues, and animal substances), forestry (such as wood resources) and related industries including fisheries and aquaculture, as well as biodegradable fraction of industrial and municipal waste, such as municipal solid waste or wastepaper. In a preferred embodiment, the biomass is straw or organic fraction of municipal solid wastes. In a more preferred embodiment, the biomass is plant biomass, more preferably selected from the list consisting of: fermentable sugar-rich biomass, such as sugarcane, starchy biomass, for example, wheat grain, or corn straw.

The recombinant beta-xylosidase enzyme produced by means of the host cell of the invention, as well as the host cell or the composition of the present invention may be used in the production of monosaccharides, disaccharides, and polysaccharides as chemical or fermentation feedstocks from biomass for the production of ethanol, plastics, or other products or intermediates.

The host cell of the present invention may be used as a source of the polypeptide having beta-xylosidase activity, and other cellulolytic enzymes, in a fermentation process with the biomass.

The predominant polysaccharide in the primary cell wall of biomass is cellulose, the second most abundant is hemi-cellulose, and the third is pectin. The secondary cell wall, produced after the cell has stopped growing, also contains polysaccharides and is strengthened through polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually bind through hydrogen bonds to cellulose, as well as to other hemicelluloses, which helps stabilize the cell wall matrix. The recombinant beta-xylosidase enzymes produced by the host cell of the invention may be used in conjunction with the other cellulolytic enzymes to further degrade the cellulose component of the biomass substrate.

The degradation or hydrolysis of biomass into fermentable sugars, process also known as "saccharification", by means of the recombinant beta-xylosidase enzyme expressed by the host cell of the invention, the host cell of the invention or the composition of the invention can be followed by a fermentation process in which the obtained fermentable sugars are used in order to finally obtain a bioproduct such as bioethanol.

Thus, another preferred embodiment of this aspect of the invention refers to the use of the recombinant beta-xylosidase enzyme produced by means of the host cell of the invention, the use of the host cell of the invention or the use of the composition of the invention for the degradation of biomass in a bioproduct production process.

The term "bioproduct" or "bio-based products" refers to those materials, chemicals and energy derived from renewable biological resources. Examples of these bioproducts are, but not limited to, hydrocarbon compounds in their different forms, such as aliphatic (saturated, insaturated, cyclic) or aromatic, as alkanes, alkenes, alkines, cyclic forms of these compounds or aromatic hydrocarbons; oxygenated substances as alcohols, ethers, aldehydes, ketones or carboxylic acids; nitrogenated substances as amines, amides, nitrocompounds or nitriles; halogenated substances as halures. The term "bioproducts" includes also any combination of the compounds described above, compounds further derived from the compounds described above by any kind of physical, chemical or biological treatment, polymers from the compounds described above, compounds described above substituted by any functional group or element in one or more of their bounds and branched forms of the compounds described above.

Furthermore, the term "bioproduct" or "bio-based products" also refers to high-value-added products which can be obtained by chemical transformation of sugars or by biological fermentation of said sugars. Examples of microorganisms which can carry out the fermentation of sugars are, without limiting to, *Bacillus thermoglucosidaisus, Clostridium butyricum, Clostridium acetobutylicum, Clostridium beijerinckii, Corynebacterium glutamicum, Enterobacter aerogenes, E. coli, Geobacillus themoglucosidasius, Klebsiella oxytoca, Lactobacillus sp. Leunoscoc mesenteroides, Thermoanaerobacter BG1L1, Thermoanaerobacter ethanolicus, Thermoanaerobacter mathranii, Thermoanaerobacter thermosaccharolyticum, Zymobacter palmae, Zymomonas mobilis Candida arabinofermentans, Candida boidinii, Candida diddensis, Candida fermentans, C. lucknowense, Candida pastoris, Candida shehatae, Candida sonorensis, Candida tropicalis, Hansenula anómala, Hansenula polymorpha, Kluyveromyces fragilis, Kluyveromyces marxianus, Pichia pastoris, Pichia stipitis, S. cerevisiae, Saccharomyces bulderi, Saccharomyces barnetti, Saccharomyces exiguus, Saccharomyces diastaticus, Saccharomyces uvarum, Schizosaccharomyces pombe, Pseudomonas sp.,* such as, *P. aeruginosa, P. putida,* or combinations thereof.

Non-limiting examples of bioproducts obtained as consequence of fermenting sugars are acids, alcohols, aromatics compounds, aldehydes, ketones, triglycerides, gases, fatty acids (such as palmitic acid, lauric acid, fatty acids-Δ2, etc.), biopolymers, proteins, peptides, amino acids, vitamins (such as B12, riboflavin, beta-carotene, etc.), antibiotics (such as penicillin, cephalosporins, tetracycline, etc.), pharmaceuticals, industrial enzymes, and combinations thereof.

Non-limiting examples of acids include acetic acid, lactic acid, propionic acid, 3-hydroxypropionic, butyric acid, gluconic acid, itaconic acid, citric acid, succinic acid, levulinic acid, glutamic acid, succinic acid, beta-cetoacid, beta-cetoalcohol, beta-hydroxiacid, and combinations thereof.

Non-limiting examples of alcohols include methanol, ethanol, propanol, hexanol, octanol, dodecanol, 1,3-butanodiol(1-3 diol), 1-alcohol, isopropanol, butanol, ethylene glycol, propanediol, butanediol, glycerol, erythritol, xylitol, sorbitol, and combinations thereof.

Non-limiting examples of amino acids include glutamic acid, aspartic acid, methionine, lysine, glycine, arginine, threonine, phenylalanine, tyrosine, and combinations thereof.

Other non-limiting examples of fermentation products include gases, such as methane, butane, ethylene, acetone, hydrogen, carbon dioxide, etc.; hydrocarbon, such as alkanes, alkenes, alkines, aromatic hydrocarbons, etc.; nitrogen compounds such as amines, amides, nitrocompounds or nitriles; halides; etc.

Ethanol can be produced by enzymatic degradation of biomass and conversion of the released saccharides to ethanol. This kind of ethanol is often referred to as bioethanol. It can be used as a fuel additive or extender in blends of from less than 1% and up to 100% (a fuel substitute).

In a more preferred embodiment the bioproduct is biofuel. The term "biofuel" as used herein refers to a hydrocarbon, or a mixture thereof, which can be used as fuel and is obtained using fermentable biomass as starting material. Examples of biofuels include, but are not limit to, ethanol or bioethanol, butanol and biodiesel. In a more preferred embodiment, the biofuel is bioethanol.

The term "bioethanol" refers to an alcohol made by fermentation, mostly from fermentable biomass, such as carbohydrates produced in sugar or starch crops such as corn or sugarcane.

Non-limiting examples of fermentation organisms and associated product include the following. Fermentation of carbohydrates to acetone, butanol and ethanol by: (i) solventogenic *Clostridia* as described by Jones and Woods (1986) Microbiol. Rev. 50:484-524; (ii) a mutant strain of *Clostridium acetobutylicum* as described in U.S. Pat. No. 5,192,673; and (iii) a mutant strain of *Clostridium beijerinckii* as described in U.S. Pat. No. 6,358,717 is known. Fermentation of carbohydrates to ethanol by modified strains of *E. coli* has been described by Underwood et al., (2002) Appl. Environ. Microbiol.68:6263-6272 and by a genetically modified strain of *Zymomonas mobilis* is described in US 2003/0162271 A1. Preparation of lactic acid by recombinant strains of *E. coli* (Zhou et al., (2003) Appl. Environ. Microbiol. 69:399-407), natural strains of *Bacillus* (US20050250192), and *Rhizopus oryzae* (Tay and Yang (2002) Biotechnol. Bioeng. 80:1-12) is known. Recombinant strains of *E. coli* have been used as biocatalysts in fermentation to produce 1,3 propanediol (U.S. Pat. Nos. 6,013,494 and 6,514,733) and adipic acid (Niu et al., (2002) Biotechnol. Prog. 18:201-211). Acetic acid has been produced using recombinant *Clostridia* (Cheryan et al., (1997) Adv. Appl. Microbiol. 43:1-33) and newly identified yeast strains (Freer (2002) World J. Microbiol. Biotechnol. 18:271-275). Production of succinic acid by recombinant *E. coli and* other bacteria is disclosed in U.S. Pat. No. 6,159,738 and by mutant recombinant *E. coli* in Lin et al., (2005) Metab. Eng. 7:116-127). Pyruvic acid has been produced by mutant *Torulopsis glabrata* yeast (Li et al., (2001) Appl. Microbiol. Technol. 55:680-685) and by mutant *E. coli* (Yokota et al., (1994) Biosci. Biotech. Biochem. 58:2164-2167). Recombinant strains of *E. coli* have been used for production of para-hydroxycinnamic acid (US20030170834) and quinic acid (US20060003429).

In a fifth aspect, the present invention refers to a method of producing fermentable sugars, hereinafter "first method of the invention", comprising:
a) Incubating biomass, preferably pretreated biomass, with the host cell of the invention, the recombinant beta-xylosidase enzyme produced by means of the host cell of the invention or with the composition of the invention, and
b) Recovering the fermentable sugars obtained after the incubation in step (a).

A pretreatment process of the biomass is often required for increasing the access of the enzymes to their substrates and consequent efficient hydrolysis. Pretreatment uses various techniques, including but not limited to ammonia fiber explosion, chemical treatment and steam explosion at high temperatures to alter the structure of cellulosic biomass and make cellulose more accessible. The use of the host cell of the invention, the recombinant beta-xylosidase enzyme produced by means of the host cell of the invention or the composition of the invention in the methods of the present invention is advantageous since high temperatures are not required in the pretreatment process of the biomass.

The term "fermentable sugar", as used herein, refers to simple sugars, such as glucose, xylose, arabinose, galactose, manose, rhanmose, sucrose or fructose, among others.

A sixth aspect of the present invention refers to a method of producing a bioproduct from biomass, hereinafter "second method of the invention", comprising:
a) Incubating biomass, preferably pretreated biomass, with the host cell of the invention, the recombinant beta-xylosidase enzyme produced by means of the host cell of the invention or with the composition of the invention,
b) Fermenting the fermentable sugars obtained after the incubation of step (a) with at least one fermenting microorganism, and
c) Recovering the bioproduct obtained after the fermentation in step (b).

The term "fermenting or fermentation" as used herein, refers to a biological transformation process caused by the activity of some microorganisms in which sugars such as glucose, fructose, and sucrose are converted into ethanol. The microorganisms used therefore, are fermenting microorganisms, which have a fermentation capacity, such as yeasts, preferably *S. cerevisiae.*

The term "recovery" as used herein, refers to the collection of fermentable sugars obtained after the incubation in step (a) of the first method of the invention or bioproduct obtained after fermentation of step (b) of the second method of the invention. The recovery may occur by any method known in the art, including mechanical or manual ones.

In a preferred embodiment of the second method of the invention, the bioproduct is biofuel, more preferably bioethanol.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Fig. 1****. Expression vector pBase-5K-4.** Expression vector with *Pcbh1* as promoter, *Tcbh1* as terminator sequences and *pyr4* as selection marker.
**Fig. 2****. Expresion plasmid pABC656.** This plasmid allows the expression of native *Anbxl* cDNA from *A. nidulans* with glucoamylase A signal peptide (SPGA) from *A.awamori.*
**Fig. 3****. Xylose production profiles during the enzymatic hydrolysis of biomass by the enzymatic mixtures produced by *M. thermophila* C1 and transformants expressing SPGA-AnBxl.** Xylose yield is calculated as the percentage of xylose released compared to the maximum (%), according to the analysis of pre-treated material. Data represent the average of three independent samples, and bars indicate the standard deviation. Control cocktail: cocktail produced by *M. thermophila* C1 parent strain; Cocktails 1-5: cocktails produced by *M. thermophila* C1 strain successfully expressing SPGA-AnBxl.
**Fig. 4****. Xylobiose consumption profiles during the enzymatic hydrolysis of biomass by the enzymatic mixtures produced by *M. thermophila* C1 and transformants expressing SPGA-AnBxl.** Xylobiose consumption is calculated as the percentage of xylobiose hydrolysated compared to the value of control enzyme cocktail (expressed as 100%). Data represent the average of three independent samples, and bars indicate the standard deviation. Control cocktail: cocktail produced by *M. thermophila* C1 parent strain; Cocktails 1-5: cocktails produced by *M. thermophila* C1 strain successfully expressing SPGA-AnBxl.
**Fig. 5****. Comparative xylose release percentage by the host cell of the invention expressing different betaxylosidases.**
   SPGA-AnBxl: *M. thermophila* C1 cell expressing the mature beta-xylosidase enzyme from *A. nidulans* (SEQ ID NO: 1) with the signal peptide of glucoamylase A from *A. awamori* (SEQ ID NO: 2). SPGA-HiBxl: *M. thermophila* C1 cell expressing the mature beta-xylosidase enzyme from *Humicola insolens* (SEQ ID NO: 8) with the signal peptide of glucoamylase A from *A. awamori* (SEQ ID NO: 2), resulting in SEQ ID NO: 10.

### Examples

### Example 1. Expression of beta-xylosidase AnBxl from A. nidulans with signal peptide of glucoamylase A from A.awamori, in M. thermophila C1. Construction of the expression vector and beta-xylosidase activity analysis in M. thermophila transformants.

*M. thermophila* C1 is a good host for expressing and secreting heterologous proteins and polypeptides. The beta-xylosidase gene *anbxl* (AN8401.2, accession number: Q5ATH9) from *A. nidulans* was used to express the enzyme and test its enzymatic performance in the present invention.

The *anbxl* cDNA sequence was synthesized *in vitro* after optimization, leading to remove the recognition sites for the most common restriction enzymes without altering the amino acid sequence. The cDNA nucleotide sequence of *anbxl* and the deduced amino acid sequence are shown in SEQ ID NO: 4 and SEQ ID NO: 1 respectively. The coding sequence is 2289 in length by including the stop codon. The encoded predicted protein is 763 amino acids long with a predicted molecular mass of 82.2 KDa and an isoelectric point of 4.52 Using the Signal IP program (Petersen et al., 2011, Signal IP 4.0, Nature Methods, 8:785-786), a signal peptide of 23 residues was predicted. The predicted mature protein of SEQ ID NO: 3 contains 740 amino acids with a predicted molecular mass of 79.6 KDa and an isoelectric point of 4.50 (data obtained using ProtParam Tool (Gasteiger E., *et al,* 2005))

Before synthesis *in vitro* the signal peptide from AnBxl native protein was replaced to increase secretion of AnBxl mature protein in *M. thermophila.* Native signal peptide from AnBxl was substituted by the signal peptide of glucoamylase A from *Aspergillus awamori* shown in SEQ ID NO: 2.

The gene *anbxl* was synthesized *in vitro* in the plasmid pBase-5K-4, which contains the promoter sequence of cellobiohydrolase 1 gene (*Pcbh1*), corresponding to an upstream region of 1796 bp of the cellobiohydrolase 1 gene (*cbh1,* NCBI *Accession number* XP_003660789.1) of *M. thermophila* C1. The expression vector pBase-5K-4 also contained the terminator sequence of the cellobiohydrolase 1 gene from *M. thermophila* C1 (T*cbh1,* corresponding to a downstream region of 1014 bp of *cbh1*). The pBase 5K-4 plasmid also contains *pyr4* gene (NCBI Accession number XP_003660657.1) from the same strain as selection marker. The *pyr4* gene encodes for a functional orotidine-5'-monophosphate decarboxylase and its expression allows complementation of the uridine auxotrophy in the corresponding auxotrophic *M. thermophila* C1 host strain (*pyr4⁻*). The map of the expression vector pBase-5K-4 is shown in Figure 1.

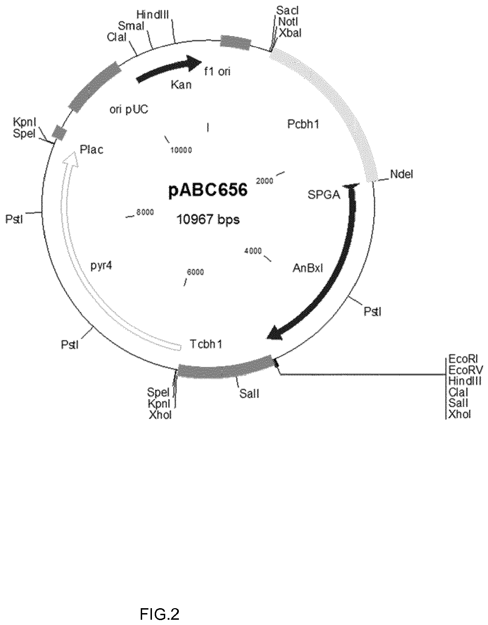

The expression vector pBase-5K-4 with synthetized SPGA*-anbxl gene* was transformed in XL1Blue MRF *E. coli* electro-competent cells following the protocol provided by the manufacturer (Stratagene). The recombinant plasmid obtained was named pABC656 and is shown in Figure 2.

The plasmid pABC656 containing *anbxl* from *A. nidulans* under P*cbh1* promoter sequence and *pyr4* as selection marker, was transformed in the *M. thermophila C1 pyr4* derivative (Verdoes et al., 2007, Ind. Biotechnol. 3 (1)), auxotrophic host strain previously used in other high-throughput screening in *M. thermophila.* The DNA was introduced in the host strain using protoplast transformation (US7399627B2). The transformants were plated out in agar plates without uridine supplementation. After 5 days of incubation at 35 °C, resulting prototrophic transformants (expressing *pyr4* gene) were analysed.

### Example 2. Beta-xylosidase activity determination on enzymatic mixtures produced by M. thermophila C1 and transformants expressing the SPGA-AnBxl.

The SPGA-AnBxl transformants were inoculated in 96-well microtitter plates (MTPs) cultures to identify beta-xylosidase activity in transformants expressing *anbxl.* Hydrolytic activity on pNXP (Sigma N2132) as substrate was measured.

Most of the transformants tested showed an increase of beta-xylosidase activity compared to untransformed *M. thermophila* C1 control. All the transformants with beta-xylosidase activity were confirmed in a second round test as defined in US7794962B2. Some of the positive transformants were confirmed at flask scale (Verdoes et al., 2007, Ind. Biotechnol. 3 (1); Visser et al., 2011, Ind. Biotechnol. 7 (3)) and beta-xylosidase activity was measured from culture supernatants. Six different enzymatic cocktails were produced (see Table 1): a control from parent strain (control cocktail) and five transformants expressing SPGA-AnBxl (Enzyme cocktails 1-5). The control cocktail consisted of the mixture of extracellular enzymes produced by *M. thermophila* C1 strain under the production conditions described in the references given above. The SPGA-AnBxl enzyme cocktails (cocktails 1-5) consisted of the mixtures of enzymes produced by this C1 strain successfully expressing pABC656 (described in example 1) under identical production conditions.

**Table 1. Specific activity of enzymatic mixtures produced by M. thermophila C1 and transformants expressing SPGA-AnBxl. Analyses were carried out using pNXP as substrate. Errors are indicated as the standard deviation (SD) of three independent measurements.**

| **Enzyme cocktail** | **BXL activity (U mg prot.⁻¹)** | **SD** |
|---|---|---|
| Control cocktail | 15.31 | 0.01 |
| 1 | 320.57 | 24.29 |
| 2 | 447.82 | 25.82 |
| 3 | 195.89 | 11.18 |
| 4 | 313.35 | 10.43 |
| 5 | 171.98 | 7.24 |

### Beta-xvlosidase activity determination

Beta-xylosidases (EC 3.2.1.27) are hydrolytic enzymes that catalyze the cleaving off the terminal xylose units from the non-reducing end of the short xylose oligomers arising from the endoxylanase (EC 3.2.1.8) activity towards xylan.

Beta-xylosidase activity was determined using *p*-nitrophenyl-beta-D-xylopyranoside (pNXP, Sigma N2132) as substrate. For this pNXP assay, the enzymatic reaction mixtures (1 mL final volume) containing 100 µmol sodium acetate buffer (pH 5.0), 100 µg pNXP (0.33 µmol) and proper amount of respective enzyme cocktail were incubated at 50 °C for 10 minutes. The amount of *p*-nitrophenol released was measured at A₄₁₀ (ε410= 15.2 mM⁻¹ cm⁻¹) after addition of 100 µg sodium carbonate to stop the reaction mixtures. One unit of pNXP hydrolysing activity was defined as the amount of enzyme needed to release 1 µmol *p*-nitrophenol per minute. The specific activities obtained are shown in Table 1.

The control cocktail exhibits significantly less pNXP activity than the cocktails produced by *M. thermophila* C1 successfully expressing pABC656 under identical production conditions.

Total protein of the enzymatic mixtures was determined by the BCA method (Applichem, A7787 0500).

### Example 3. Effect of transformants expressing SPGA-AnBxl cocktails on the release of xylose during the enzymatic hydrolysis of xylan-containing biomass.

### Enzymatic hydrolysis experiments

Unwashed pretreated corn stover (PCS) was used as substrate for enzymatic hydrolysis. Pre-treatment of the biomass was performed by a modification of the steam explosion system described by Nguyen et al., 1998, Appl. Biochem. Biotechnol. 70-72, in which no acid treatment was applied so that xylan hydrolysis was impaired. Incomplete release of xylose from pre-treated material was necessary for the evaluation of the effect of the SPGA-AnBxl activity.

The compositional analysis of this material was performed accordingly to the Standard Biomass Analytical Procedures from National Renewable Energy Laboratory (NREL) (http://www.nrel.gov /biomass/analytical_procedures.html).

Hydrolysis of the pre-treated biomass (20 grams of total reaction mass) was performed in 100 mL ISO bottles. Water was added to adjust the solid loading to 20 % total solids (based on pre-treated substrateafter the pH was adjusted to 5.5 by addition of 25% ammonia solution (NH₄OH). The enzyme loading was 12 mg protein per g glucan content of each enzymatic cocktail produced by *M. thermophila* C1 strain and AnBxl transformants. The hydrolysis was performed by incubating at 50 °C inside 25 mm orbit diameter shakers at 150 rpm for 72 hours.

Xylose release and xylobiose hydrolysis profiles obtained during the enzymatic hydrolysis are shown in Figure 3 and Figure 4, wherein it can be seen that the use of cocktails obtained by transformants expressing SPGA-AnBxl (SEQ ID NO: 4) leads to a high xylose production and xylobiose consumption as compared with the control *M. thermophila* C1 parent strain.

### Example 4. Performance of SPGA-AnBxl compared to beta-xylosidases from other organisms expressed in M. thermophila C1.

Following the conditions described at Example 3, a similar experiment was carried out in order to compare a transformant expressing SPGA-AnBxl with a transformant expressing a beta-xylosidase from *H. insolens* (*Hi*) (SPGA-HiBxl). The gene that codifies for this protein was synthetized *in vitro* and it was cloned in the same vector than *anbxl.* This enzyme did display a clear signal peptide, so glucoamylase A signal peptide from *A. awamori* was added as described for *anbxl* in Example 1.

The mature beta-xylosidase enzyme from *H. insolens* (SEQ ID NO: 8) with the signal peptide of glucoamylase A from *A. awamori* (SEQ ID NO: 2), resulting in the construct SPGA-HiBxl (SEQ ID NO: 10). Nucleotide sequences encoding mature protein of SEQ ID NO: 8 and protein SEQ ID NO: 10 are shown as SEQ ID NO: 9 and SEQ ID NO: 11.
SEQ ID NO: 8: 
MAPLITNIYTADPSAHVFNGKLYIYPSHDRETDIQFNDNGDQYDMADYHVFSLDSLDPPS
EVTDHGVVLKVEDIPWVSKQLWAPDAATKDGKYYLYFPARDKEGIFRIGVAVSDKPEGPF
TPDPEPIKGSYSIDPAVFVDDDGSAYMYFGGLWGGQLQCYQKGNNIFDAEWSGPKEPSGS
GAKALGPRVAKLTDDMRQFAEEVREIVILAPETGEPLAADDHDRRFFEAAWMHKYNGKYY
FSYSTGDTHYLVYAVGDSPYGPFTYGGRILEPVLGWTTHHSIVEFQGRWWLFHHDCELSK
GVDHLRSVKVKEIWYDKDGKIVTEKPE
SEQ ID NO: 9: 
gcccccctcatcaccaacatctacaccgccgacccctccgcccacgtcttcaacggcaagct
ctacatctacccctcccacgaccgcgagaccgacatccagttcaacgacaacggcgaccagt
acgacatggccgactaccacgtcttctccctcgactccctcgaccccccctccgaggtcacc
gaccacggcgtcgtcctcaaggtcgaggacatcccctgggtctccaagcagctctgggcccc
cgacgccgccaccaaggacggcaagtactacctctacttccccgcccgcgacaaggagggca
tcttccgcatcggcgtcgccgtctccgacaagcccgagggccccttcacccccgaccccgag
cccatcaagggctcctactccatcgaccccgccgtcttcgtcgacgacgacggctccgccta
catgtacttcggcggcctctggggcggccagctccagtgctaccagaagggcaacaacatct
tcgacgccgagtggtccggccccaaggagccctccggctccggcgccaaggccctcggcccc
cgcgtcgccaagctcaccgacgacatgcgccagttcgccgaggaggtccgcgagatcgtcat
cctcgcccccgagaccggcgagcccctcgccgccgacgaccacgaccgccgcttcttcgagg
ccgcctggatgcacaagtacaacggcaagtactacttctcctactccaccggcgacacccac
tacctcgtctacgccgtcggcgactcccctacggccccttcacctacggcggccgcatcct
cgagcccgtcctcggctggaccacccaccactccatcgtcgagttccagggccgctggtggc
tcttccaccacgactgcgagctctccaagggcgtcgaccacctccgctccgtcaaggtcaag
gagatctggtacgacaaggacggcaagatcgtcaccgagaagcccgagtaa
SEQ ID NO: 10: 
MSFRSLLALSGLVCSGLAAPLITNIYTADPSAHVFNGKLYIYPSHDRETDIQFNDNGDQY
DMADYHVFSLDSLDPPSEVTDHGVVLKVEDIPWVSKQLWAPDAATKDGKYYLYFPARDKE
GIFRIGVAVSDKPEGPFTPDPEPIKGSYSIDPAVFVDDDGSAYMYFGGLWGGQLQCYQKG
NNIFDAEWSGPKEPSGSGAKALGPRVAKLTDDMRQFAEEVREIVILAPETGEPLAADDHD
RRFFEAAWMHKYNGKYYFSYSTGDTHYLVYAVGDSPYGPFTYGGRILEPVLGWTTHHSIV
 EFQGRWWLFHHDCELSKGVDHLRSVKVKEIWYDKDGKIVTEKPE
SEQ ID NO: 11:

ATGTCGTTCCGATCTCTTCTCGCCCTGAGCGGCCTTGTCTGCTCGGGGTTGGCAGCCCCC
CTCATCACCAACATCTACACCGCCGACCCCTCCGCCCACGTCTTCAACGGCAAGCTCTAC
ATCTACCCCTCCCACGACCGCGAGACCGACATCCAGTTCAACGACAACGGCGACCAGTAC
GACATGGCCGACTACCACGTCTTCTCCCTCGACTCCCTCGACCCCCCCTCCGAGGTCACC
GACCACGGCGTCGTCCTCAAGGTCGAGGACATCCCCTGGGTCTCCAAGCAGCTCTGGGCC
CCCGACGCCGCCACCAAGGACGGCAAGTACTACCTCTACTTCCCCGCCCGCGACAAGGAG
GGCATCTTCCGCATCGGCGTCGCCGTCTCCGACAAGCCCGAGGGCCCCTTCACCCCCGAC
CCCGAGCCCATCAAGGGCTCCTACTCCATCGACCCCGCCGTCTTCGTGGACGACGACGGC
TCCGCCTACATGTACTTCGGCGGCCTCTGGGGCGGCCAGCTCCAGTGCTACCAGAAGGGC
AACAACATCTTCGACGCCGAGTGGTCCGGCCCCAAGGAGCCCTCCGGCTCCGGCGCCAAG
GCCCTCGGCCCCCGCGTCGCCAAGCTCACCGACGACATGCGCCAGTTCGCCGAGGAGGTC
CGCGAGATCGTCATCCTCGCCCCCGAGACCGGCGAGCCCCTCGCCGCCGACGACCACGAC
CGCCGCTTCTTCGAGGCCGCCTGGATGCACAAGTACAACGGCAAGTACTACTTCTCCTAC
TCCACCGGCGACACCCACTACCTCGTCTACGCCGTCGGCGACTCCCCCTACGGCCCCTTC
ACCTACGGCGGGCGCATCCTGGAGCCCGTCCTCGGCTGGACCACCCACCACTCCATCGTC
GAGTTCCAGGGCCGCTGGTGGCTCTTCCACCACGACTGCGAGCTGTCCAAGGGCGTGGAC
CACCTCCGCTCCGTCAAGGTCAAGGAGATCTGGTACGACAAGGACGGCAAGATCGTCACC
GAGAAGCCCGAGTAA

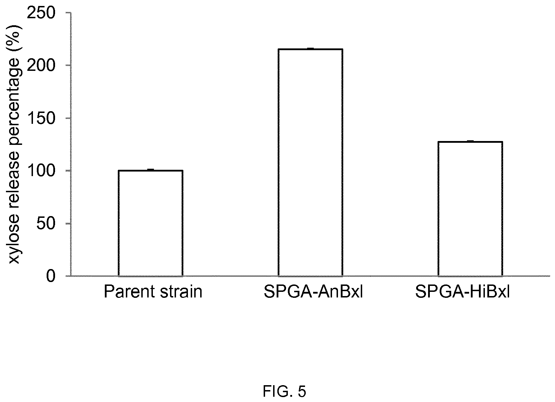

As shown in Figure 5, xylose release is higher by transformant expressing SPGA-AnBxl than the one expressing SPGA-HiBxl.

## Claims

1. A *Myceliophthora thermophila* cell comprising a nucleotide sequence encoding a recombinant beta-xylosidase enzyme comprising an amino acid sequence having at least 70% sequence identity to SEQ ID NO: 1.

2. The cell according to claim 1, wherein the recombinant beta-xylosidase enzyme comprises an amino acid sequence having at least 80% sequence identity to SEQ ID NO: 1.

3. The cell according to claim 2, wherein the recombinant beta-xylosidase enzyme comprises an amino acid sequence having at least 90% sequence identity to SEQ ID NO: 1.

4. The cell according to claim 3, wherein the recombinant beta-xylosidase enzyme comprises the amino acid sequence SEQ ID NO: 1.

5. The cell according to any of claims 1 to 4, wherein the recombinant beta-xylosidase enzyme comprises a signal peptide.

6. The cell according to claim 5, wherein the signal peptide is the glucoamylase A signal peptide, preferably, the glucoamylase A signal peptide from *Aspergillus awamori.*

7. The cell according to claim 6, wherein the glucoamylase A signal peptide from *Aspergillus awamori* comprises the amino acid sequence SEQ ID NO: 2.

8. The cell according to claim 7, wherein the recombinant beta-xylosidase enzyme comprises the amino acid sequence SEQ ID NO: 3.

9. The cell according to any of claims 1 to 8, wherein the nucleotide sequence comprises the sequence SEQ ID NO: 4.

10. The cell according to claim 9, wherein the nucleotide sequence is bound to a nucleotide sequence encoding a signal peptide, preferably, the nucleotide sequence encoding the signal peptide of the glucoamylase A, preferably, the nucleotide sequence is the glucoamylase A signal peptide from *Aspergillus awamori,* more preferably, the nucleotide sequence is the glucoamylase A signal peptide from *Aspergillus awamori* of SEQ ID NO: 5.

11. The cell according to claim 10, wherein the nucleotide sequence comprises the sequence SEQ ID NO: 6.

12. A recombinant beta-xylosidase enzyme expressed by the host cell of any of claims 1 to 11.

13. A composition comprising the host cell according to any of claims 1 to 11 and/or the recombinant beta-xylosidase enzyme according to claim 12.

14. A composition which is an enzymatic mixture obtained by the cell of any of claims 1 to 11.

15. The composition according to claim 14, further comprising other cellulolytic enzymes derived from the host cell according to any of claims 1 to 3.

16. Use of the host cell according to any of claims 1 to 11, the recombinant beta-xylosidase enzyme according to claim 12, or the composition according to any of claims 13 to 15, for the degradation of biomass.

17. Use according to claim 16 for the degradation of biomass in a bioproduct production process.

18. Use according to claim 17 wherein the bioproduct is biofuel.

19. Use according to claim 18, wherein the biofuel is bioethanol.

20. A method of producing fermentable sugars comprising:
a. Incubating biomass with the recombinant beta-xylosidase enzyme according to claim 12 or the composition according to any of claims 13 to 15, and
b. Recovering the fermentable sugars obtained after the incubation in step (a).

21. A method of producing a bioproduct from biomass comprising:
a. Incubating biomass with the recombinant beta-xylosidase enzyme according to claim 12 or the composition according to any of claims 13 to 15,
b. Fermenting the fermentable sugars obtained after the incubation of step (a) with at least one fermenting microorganism, and
c. Recovering the bioproduct obtained after the fermentation in step (b).

22. The method according to claim 21, wherein the bioproduct is biofuel.

23. The method according to claim 22, wherein the biofuel is bioethanol.
